(19) Europäisches Patentamt
European Patent Office
Office européen des brevets

(11) EP 1 213 351 A2

(12) EUROPEAN PATENT APPLICATION

(43) Date of publication:
12.06.2002 Bulletin 2002/24

(21) Application number: 01308694.7

(22) Date of filing: 12.10.2001

(51) Int Cl.[7]: **C12N 15/11**, C12N 9/00, C07H 21/02, A61K 48/00, A61K 31/713, C12N 1/21, C12N 5/10, C12Q 1/68 // A61P31/18, A61P35/00

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.10.2000 JP 2000313320**

(71) Applicant: **National Institute of Advanced Industrial Science and Technology**
**Tokyo 100-8921 (JP)**

(72) Inventors:
- **Taira, Kazunari**
**Tsukuba-shi, Ibaraki 305-0046 (JP)**
- **Warashina, Masaki,**
**Nat.Inst.Adv.Ind.Science+Techn.**
**Tsukuba-shi, Ibaraki 305-8561 (JP)**
- **Warashina, Tomoko,**
**Nat.Inst.Advanced Ind.Sci+Techn**
**Tsukuba-shi, Ibaraki 305-8561 (JP)**

(74) Representative: **Maschio, Antonio**
**D Young & Co,**
**21 New Fetter Lane**
**London EC4A 1DA (GB)**

(54) **Nucleic acid enzymes acquiring an activity for cleaving a target RNA by recognising another molecule**

(57) The present invention relates to a nucleic acid enzyme which has a sensor site and a cleavage active site, wherein the nucleic acid enzyme exhibits a cleavage activity towards a target RNA only when the target RNA binds to the cleavage active site while another RNA, DNA or protein binds to the sensor site, and use of the enzyme. According to the present invention, the nucleic acid enzyme has an RNA-cleaving activity by which harmful cells such as infected or cancerated cells can be killed selectively and efficiently. Furthermore, the nucleic acid enzyme has an RNA-cleaving ability that can be regulated by a cell-specifically or time-specifically expressed RNA, DNA or protein or by an artificially introduced RNA, DNA or protein.

EP 1 213 351 A2

**Description**

FIELD OF THE INVENTION

The present invention relates to a nucleic acid enzyme and use thereof. More specifically, the present invention relates to a nucleic acid enzyme that acquires an activity for cleaving a target RNA by complementarily binding with a co-existing molecule such as RNA, DNA or protein, for example a nucleic acid enzyme which possesses an ability to cleave any RNA and is capable of selectively and efficiently killing harmful cells such as infected or cancerated cells, or a nucleic acid enzyme having an RNA cleavage activity which can be regulated by a cell-specifically or time-specifically expressed RNA, DNA or protein or by an artificially introduced RNA, DNA or protein, and to use of the enzyme.

BACKGROUND OF THE INVENTION

At the beginning of 1980, the ribozyme (ribonucleotide acid + enzyme) which is an RNA having a catalytic function, was found by the discovery of a self-splicing phenomenon in tetrahymena rRNA (K. Kruger, P. J. Grabowski, A. J. Zaug, J. Sands, D. E. Gottschling, T. R. Cech, Cell, 31, 147-157(1982)), and by the analysis of ribonuclease P which is a complex enzyme of RNA and protein (C. Guerrier-Takada, K. Gaydiner, T. Marsh, N. Pace, S. Altman, Cell, 35, 849-857(1983)). Thereafter, various ribozymes have been discovered (R. H. Symons, Trend. Biochem. Sci., 14, 445-450 (1989); R. H. Symons, Annu. Rev. Biochem., 61, 641-671 (1992); J. Bratty, P. Chartrand, G. Ferbeyre, R. Cedergren, Biochim. Biophys. Acta, 1216, 345-359(1993); Hasehoff. J and W. L. Gerlach, Nature, 334, 585-591 (1988); C. J. Hutchins, P. D. Rathjen, A. C. Forster, R. H. Symons, Nucleic Acids. Res., 14, 3627-3640 (1986)). As in the discovery of the reverse transcriptase, the intron and the RNA editing, the discovery of the ribozyme shook the idea of an established central dogma. At the same time, RNA which had not been recognized as anything more an informational intermcdiary, began, due to its two roles i.e. (genetic) information and (catalytic) function, to attract attention as a central molecule in the "RNA world" hypothesis, which places the RNA molecule as the true origin of life (G. F. Joyce, Nature, 338, 217-224 (1989); N. R. Pace, T. L. Marsh, Origins of Life, 16, 97(1985); A. Lazcano, R. Guerrero, J. Oro, J. Mol. Evol., 27, 283 (1988); L. E. Orgel, Nature, 358, 203(1992); R. F. Gesteland, J. F. Atkins, The RNA World, Monograph 24, Cold Spring Harbor Laboratory Press, Plainview, New York (1993)).

The hammerhead ribozyme is one of the most well studied ribozymes until now (Figure 1A, left). This is a ribozyme which functions in nature as a self-cleaving reaction (a cis-form) (T. R. Cech, Annu. Rev. Biochem., 59, 543 (1990); A. C. Foster, R. H. Symons, Cell, 49, 211 (1987); A. C. Jeffries, R. H. Symons, Nucleic Acids Res., 17, 1371 (1989)), and as the result that the ribozyme was divided into two strands of RNA (i.e., a substrate binding region and an enzyme activity retaining region) by Uhlenbeck et al. and Haseloff and Gerlach et al (O. C. Uhlenbeck, Nature, 328, 596(1987); J. Hasehoff, W. L. Gerlach, Nature, 334, 585 (1988), the possibility of applying ribozymes to gene therapy was suggested. Thereafter, a large number of applied researches which targeted cancers, AIDS, etc., have been reported (M. Cotten, M. L. Bimstiel, EMBO J8, 861 (1989); N. Sarver, E. Cantin, O. Chang, O. Lande, D. Stephens, J. Zaia, J. Rossi, Science 247, 1222 (1990); M. Homann, M. Tzortzakari, K. Rittner, S. Sczakiel, M. Tabler, Nucleic Acids Res 21, 2809 (1993); R. C. Mulligan, Science 0, 926 (1993); S. Altman: Proc. Natl. Acad. Sci. USA, 90. 10898 (1993); P. Marschall, J. B. Thompson, F. Eckstein, Cell. Mol. Neurobiol., 14, 523 (1994); S. M. Sullivan, J. Invest. Dermatol., 103, 85 (1994); F. H. Cameron, P. A. Jennings, Antisense Res, Dev., 4, 87 (1994); L. Q. Sun, D. Warrilow, L. Wang, C. Witherington, J. Macpherson, G. Symonds, Proc. Natl. Acad. Sci. USA, 91, 9715 (1994); R. E. Christoffersen, J. J. Marr, J. Med. Chem. 38, 2023 (1995); G. Ferbeyre, J. Bratty, H. Chen, R. Cedergern, Gene 155, 45(1995) ; M.Kiehntopf, E.L.Eaquivel, M.A.Brach, F.Herrmann, J.Mol. Med., 73, 65(1995); J. D. Thompson, D. Macejak, L. Couture, D. T. Stinchcomb, Nat. Med. 1, 277 (1995); T. Tuschl, J. B. Thomson, F. Eckstein, Curr. Opin. Struct. Biol. 5, 296 (1995)).

A ribozyme binds to a substrate RNA by forming a complementary base pair with the substrate. Thereafter, in the presence of a magnesium ion which is essential for the reaction, cleavage of the substrate RNA molecule occurs. As a substrate is recognized by a ribozyme via formation of appropriate base pairs between substrate-binding regions of Stem I and Stem III of the ribozyme and substrate sequences corresponding to them, the substrate specificity of the ribozyme is very high. With very high substrate specificity, any side effect hardly occurs in a cell, and consequently this will provide a considerable benefit when ribozyme is used as a gene expression inhibitor.

However, there are exceptions to this ribozyme's high substrate specificity. This is the case where target substrate is a chimera (where two or more different genetic sequences bind together and function as a gene). Where a sequence (e.g., exon 3) should normally be connected to another sequence (e.g., exon 2) but, as a result of splicing mistakes or the like, an incorrect chimera sequence (e.g., exon 1 - exon 3) is erroneously generated and leads to diseases such as cancers, a gene therapy, which comprises controlling the expression of this abnormal RNA with a ribozyme, may be contemplated for the purpose of treatment of the diseases. Since each sequence itself (in this example, exon 1, exon 2 or exon 3) is a normal message, it becomes important to specifically cleave only mRNA having an abnormal junction sequence (exon 1-exon 3) and thereby to suppress its expression. That is, a ribozyme having absolutely no effect on normal mRNA (exon 2 - exon 3) must be employed. (e.g. BCR-ABL mRNA, Figure 2).

When such a control of gene expression is attempted using a conventional hammerhead ribozyme, and where there exists a GUC triplet (generally, NUX (N; A, G, C or U; X: A, C or U)) which is a substrate sequence capable of being cleaved at an exon 1 - exon 3 junction site which is a sequence portion specific to abnormal mRNA, no problems arise. However, it is rare that a ribozyme cleavage sequence will luckily be found at a junction site or in the vicinity thereof. When there is no cleavable sequence at a junction site, there is no option but to target a cleavage sequence at a site far removed from the junction site. However, sequences of cleavage sites themselves, which are at a great distance from a junction, are present in both normal mRNA and abnormal mRNA. In the end, it becomes impossible to avoid the occurrence of non-specific cleavage in normal mRNA (e.g. cleavage of ABL mRNA, Figure 2). Further, even if there is a NUX sequence, being a ribozyme cleavage sequence, at a junction site or in the vicinity thereof, the probability that this is a triplet (GUC triplet) of a sequence which a hammerhead ribozyme can willingly and efficiently cleave, is even lower. Therefore, even in a case such as this, it is difficult to construct a ribozyme with extremely high specificity while maintaining high cleavage activity.

In fact, as a famous example wherein the above-mentioned chimeric mRNA causes a disease, there is the formation of Philadelphia chromosome which is responsible for causing CML (chronic myelocytic leukemia) and ALL (acute lymphocytic leukemia). In these types of leukemia, chromosomal reciprocal translocation t (9;22)(q34;q11) occurs and BCR-ABL fusion gene is formed. In CML, by two types of chromosomal reciprocal translocation, K28 translocation and L6 translocation, two types of BCR-ABL fusion genes are produced and finally two types of chimera mRNAs are formed as a result of splicing (i.e., K28 junction (b3a2) mRNA and L6 junction (b2a2) mRNA) (Figure 2). In one of them, there is a (GUU triplet) sequence near BCR-ABL junction site, capable of being cleaved by a hammerhead ribozyme (i.e., in K28 junction (b3a2) mRNA). However, in the other type of mRNA there exists no useful cleavage sequence in the vicinity of the junction site (i.e., in L6 junction (b2a2) mRNA). Because of this, it is not possible to specifically inhibit the expression from the latter mRNA using any conventional hammerhead ribozyme. Almost all attempts to inhibit the expression of an abnormal protein $p210^{BCR-ABL}$ from L6 mRNA using a ribozyme that have been reported until now involved adding a long antisense portion to the hammerhead ribozyme and allowing this portion to complementarily bind with a junction site in an attempt to impart cleavage specificity.

However, as the substrate binding portion of a ribozyme becomes longer by addition of such a long antisense portion, the step of dissociating the ribozyme from the substrate becomes much slower after the ribozyme binds to a substrate. For this reason, the ribozyme is unable to turn over as an enzyme, and its cleavage efficiency falls. Further, having gone so far to impart substrate specificity, this substrate specificity is not as high as expected. This is because the sequence of the overly long antisense portion partially binds to normal mRNA such as ABL mRNA or BCR mRNA, which undermines the ribozyme's substrate recognizing ability. As a result, non-specific cleavage of normal mRNA remains unavoidable (Figure 2).

As one solution, the present inventors have previously constructed a nucleic acid enzyme indicating allosteric cleavage activity towards substrates (PCT Publication No. WO 99/46388). That is, the inventors constructed a nucleic acid enzyme (i.e., a ribozyme) having one active center region and two substrate binding regions, which binds to L6 (b2a2) chimeric mRNA junction site at one substrate binding region, and at the other substrate binding region binds to a far removed cleavage sequence having good cleavage efficiency, and which cleaves the substrate at the site just after this cleavage sequence. Such a nucleic acid enzyme is useful where the target RNA is a disease-causing mRNA or the like.

However, because the techniques described above are limited to the occurrence of specific sequence recognition and enzyme activity in the same RNA molecule, there is the problem that it cannot be determined whether intermolecular recognition and expression of enzyme activity are possible.

An object of the present invention is to provide a nucleic acid enzyme which acquires a cleavage activity towards a specific target RNA by recognizing a co-existing RNA, DNA or protein molecule. Such an enzyme is referred to herein as a trans-maxizyme.

Another object of the present invention is to provide an expression vector comprising DNA encoding the above-mentioned nucleic acid enzyme, or a gene-transfer vehicle comprising the above-mentioned nucleic acid enzyme.

A further object of the present invention is to provide a pharmaceutical composition comprising, as an active ingredient, the above-mentioned nucleic acid enzyme, the expression vector, or the gene-transfer vehicle.

A yet further object of the present invention is to provide a method of producing the above-mentioned nucleic acid enzyme, and any use of the nucleic acid enzyme.

## SUMMARY OF THE INVENTION

Under the above-mentioned circumstances, the present inventors proceeded with research based on the idea that if it was possible to cleave an RNA molecule by recognizing one of two types of RNA molecules, then it could be possible, in the treatment of diseases including virak infections and cancers, to selectively kill only infected or cancerated cells.

To solve the above problems, the present inventors studied intensively and, as a result, completed the invention as described below.

That is, in an aspect of the present invention, there is provided a nucleic acid enzyme which, by recognizing a co-existing RNA, DNA or protein molecule, acquires cleavage activity towards another specific target RNA. Specifically, the present invention provides a nucleic acid enzyme having a sensor site and a cleavage active site, wherein the cleavage active site binds with a target RNA, and the enzyme exhibits cleavage activity towards the target RNA only when the sensor site binds with an RNA, DNA or protein different from the target RNA. As used herein, the term "sensor site" refers to a site having affinity with or binding to any sequence of an RNA, DNA or protein different from the target RNA. This site preferably comprises a sequence complementary to a nucleic acid sequence of said RNA or DNA.

The nucleic acid enzyme of the present invention can be either a monomer or a (homo or hetero) dimer, preferably a heterodimer. The nucleic acid enzyme of the present invention enables specific cleavage of a target RNA by changing its conformation such that said enzyme forms, intermolecutarly (or intramolecularly), a cavity which captures a $Mg^{2+}$ ion, when the target RNA and the RNA, DNA or protein different from the target RNA bind correctly to a sensor site and a cleavage active site of the enzyme, respectively. Because of this, it is preferred that the nucleic acid sequences of the sensor site and cleavage active site of the enzyme of the present invention are completely or substantially complementary to RNA sequences which bind to them. The term "substantially" as used herein means that the sequence of the enzyme is complementary to the RNA or DNA sequence which binds to the enzyme, to the extent that they can form a cavity which captures a magnesium ion.

The nucleic acid enzyme of the present invention can have a dimeric structure which is formed from an RNA molecule comprising nucleotide sequence (10) below and an RNA molecule comprising nucleotide sequence (20) below:

$$5'X^1_l \cdots X^1_h \, Y^1_l \cdots Y^1_i \, Z^1_l \cdots Z^1_j \, 3' \qquad (10);$$

and

$$5'Z^2_l \cdots Z^2_n \, Y^2_l \cdots Y^2_m \, X^2_l \cdots X^2_k \, 3' \qquad (20),$$

wherein $X^1_l$ to $X^1_h$, $X^2_l$ to $X^2_k$, $Y^1_l$ to $Y^1_i$, $Y^2_1$ to $Y^2_m$, $Z^1_l$ to $Z^1_j$ and $Z^2_l$ to $Z^2_n$ are each independently any one of A, U, T, C or G,

h and k are each an integer of 1 or greater (e.g. an integer from 1 to 100),
i and m are each an integer of 1 or greater (e.g. an integer from 1 to 100),
j is an integer of 1 or greater (e.g. an integer from 1 to 100),
n is an integer of 1 or greater (e.g. an integer from 1 to 100),
$X^1_l \cdots X^1_h$ and $X^2_l \cdots X^2_k$ are nucleolide sequences capable of forming a sensor site,
$Y^1_l \cdots Y^1_i$ and $Y^2_l \cdots Y^2_m$ are nucleotide sequences capable of forming a stem, comprising regions which are able to form a cavity to stably capture a $Mg^{2+}$ ion only when said RNA, DNA or protein binds to a sensor site, and
$Z^1_l \cdots Z^1_j$ and $Z^2_l \cdots Z^2_n$ are nucleotide sequences comprising regions complementary to target RNA sequences adjacent to a target RNA cleavage site.

Examples of target RNA include mRNAs encoding proteins indispensable for maintaining life of a cell (in other words, necessary for a cell's survival) or viral RNAs, or mRNAs encoding proteins associated with diseases such as cancers and viral infections. Examples include the Bcl-2 family (bcl-2, mcl-1, bfl-1, box-$\alpha$, bak, bik, bcl-$X_L$ and bcl-$X_S$) which are apoptosis preventing factors. (The wild-type hammerhead ribozyme which cleaves bcl-2 mRNA is indicated in figure 3A.) On the other hand, as an RNA, DNA or protein which co-exists with target RNA and is recognized by a trans-maxizyme sensor sequence, disease-associated RNA (preferably mRNA), DNA or protein is suitable. One example is tat mRNA or tat protein of HIV-1, a virus responsible for causing AIDS. This example of the construction of a transmaxizyme is shown in Figure 3B. A nucleic acid enzyme in this case can be, for example, an enzyme having a dimeric structure which is formed from an RNA molecule comprising nucleotide sequence (1) below and an RNA molecule comprising nucleotide sequence (2) below (see Figure 3B):

5' GGUCCUGGCC UGAUGAGAGU GAUGAGCUCU UC 3' (1) (SEQ ID NO:1);

and

5' GUCUGACUGU UCAUCGAAAC CGGGUCC 3' (2) (SEQ ID NO:2),

wherein, nucleotide Nos. 1 to 9 of nucleotide sequence (1) and nucleotide Nos. 19 to 27 of nucleotide sequence (2) may or may not be modified such that they are complementary to target RNA sequences adjacent to a target RNA cleavage site, and wherein nucleotide Nos. 20 to 32 of nucleotide sequence (1) and nucleotide Nos. 1 to 12 of nucleotide sequence (2) may or may not be modified such that they are complementary to a sequence within another mRNA specific to AIDS causative virus, HIV-1, or to a sequence of another site within tat mRNA of HIV-1.

Further, an example of the construction of a trans-maxizyme targeting another disease, chronic myelocytic leukemia, is shown in Figure 3C. Examples of an RNA which is recognized by a sensor sequence include an mRNA encoding a disease-associated protein, an mRNA which expresses tissue-specifically or time-specifically, and an RNA artificially introduced for the purpose of regulating nucleic acid enzyme activity. An example thereof is a disease-causing BCR-ABL mRNA. In this case the nucleic acid enzyme preferably has a dimeric structure which is formed from an RNA molecule comprising nucleotide sequence (3) below and an RNA molecule comprising nucleotide sequence (4) below (see Figure 3C):

5' GGUCCUGGCC UGAUGAGAGU UAUUGAUGGU CAG 3' (3) (SEQ ID NO:3);

and

5' GAAGGGCUUC UUUCAUCGAA ACCGGGUCC 3' (4) (SEQ ID NO:4),

wherein nucleotide Nos. 1 to 9 of nucleotide sequence (3) and nucleotide numbers 20 to 29 of nucleotide sequence (4) may or may not be modified such that they are complementary to a target RNA sequence in the vicinity of a target RNA cleavage site, and wherein nucleotide Nos. 20 to 33 of nucleotide sequence (3) and nucleotide Nos. 1 to 14 of nucleotide sequence (4) may or may not be modified such that they are complimentary to a sequence within another mRNA specific to chronic myelocytic leukemia, or a sequence of another site within BCR-ABL mRNA.

Another example of a molecule capable of being recognized by a sensor sequence of the nucleic acid enzyme of the invention, includes any DNA or protein, which can bind to the sensor site of the enzyme and change the conformation of the enzyme such that the enzyme forms a cavity or pocket which captures a magnesium ion, thereby allowing the enzyme to cleave a target RNA.

A linker sequence, a promoter sequence (e.g. $tRNA^{Val}$ promoter sequence, or pol II or pol III promoter sequence (e.g. tRNA or snRNA), or both may be added upstream of nucleic acid enzyme sequences (e.g. nucleotide sequences (1) to (4) above). The linker sequence added upstream of the nucleic acid enzyme sequence can comprise the following nucleotide sequence (5) or nucleotide sequence(6):

5' AAA 3' (5);

and

5' UUU 3' (6).

Further, the $tRNA^{Val}$ promoter sequence preferably comprises nucleotide sequence (7) below (see Figure 7A):

5' ACCGUUGGUU UCCGUAGUGU AGUGGUUAUC ACGUUCGCCU AACACGCGAA AGGUCCCCGG UUCGAAACCG GGCACUACAA AAACCAAC 3' (7) (SEQ ID NO:5).

Further, an additional sequence or a terminator sequence, or both can be added downstream of nucleic acid enzyme sequences (e.g. nucleotide sequences (1) to (4) above). The additional sequence can comprise any one of the following nucleotide sequences (8) to (10):

5' AAA 3' (8);

5' AACCGUA 3' (9);

and

5' UUUUU 3' (10).

The present invention further provides a nucleic acid enzyme wherein a delivery agent is attached to the nucleic acid enzyme sequence. The delivery agent is selected from the group consisting of, for example, peptides, peptide mimics, virus-derived proteins, cholesterols, steroids, cholesterol derivatives, fats, vitamins, biotin, folic acid, retinoic acid, proteins, ferritin, LDL, insulin, antibodies, saccharides or oligosaccharide, polyethylene glycol, and polymers or copolymers of amino acids, all of which have affinity for cells, tissues or constituents thereof which contain a target nucleic acid to be attacked by the nucleic acid enzyme of the invention. The delivery agent can be employed for delivering the nucleic acid enzyme at a target nucleic acid.

Further, the present invention provides an expression vector comprising DNA encoding the above-mentioned nucleic acid enzyme. The vector include both of a vector for synthesis of a nucleic acid enzyme and a vectors for gene transfer (or gene therapy).

Further, the present invention provides a method for producing the above-mentioned nucleic acid enzyme, wherein the method comprises transcribing into RNA using as a template an expression vector DNA comprising DNA encoding the nucleic acid enzyme.

Further the present invention provides a gene transfer vehicle comprising the nucleic acid enzyme. Examples of vehicles include positively charged lipids, particularly, positively charged liposomes (e.g. lipofection, DOTMA, DOSPA, and positively charged cholesterol, etc.). In this case, the vehicle is used in place of a gene transfer vector, however, chemical modification may also be applied to the trans-maxizyme RNA, which is a nucleic acid enzyme of the present invention, in order to impart resistance to any RNAase *in vivo.* Methods of introducing genes into cells by means of positively charged lipids are described in, for example, *Proteins*, *Nucleic Acids*, *Enzymes* 44 (II), 1590-1596, 1999 (Japan).

Further, the present invention provides a pharmaceutical composition comprising as an active ingredient, the above-mentioned nucleic acid enzyme, an expression vector comprising DNA encoding the nucleic acid enzyme, or a positively charge liposome. This pharmaceutical composition can be used for prevention and/or treatment of a disease caused by a target RNA which is a causative factor. Examples of a target gene include apoptosis-preventing factors such as the above-mentioned bcl-2, and examples of a co-existing recognized RNA include mRNA encoding tat protein of HIV (AIDS) and mRNAs encoding specific surface antigens of various types of cancers. The pharmaceutical composition according to the present invention in this case enables selective killing of only harmful cells such as cells infected with a virus like HIV, or cancer cells.

Further, the present invention provides a host cell which comprises the above defined nucleic acid enzyme, wherein the host cell is selected from prokaryotic cells such as *E. coli* and eukaryotic cells such as human cells, mammal cells, plant cells, yeast cells and the like.

The present invention further provides a method of specifically cleaving a target RNA *in vivo*, *in vitro* or *ex vivo*, using the above-mentioned nucleic acid enzyme. Here, in the case of the *in vivo* or *ex vivo* method, animals (particularly, mammals) and plants can be subjects. Examples of a target RNA include abnormal mRNAs which are causative factors associated with diseases.

The present invention further provides a diagnostic agent which comprises the above-defined nucleic acid enzyme. Since the nucleic acid enzyme of the present invention is inactive to normal cells but can induce apoptosis of harmful cells, this diagnostic agent can be used in the diagnosis of viral infections including AIDS, and cancers.

BRIEF DESCRIPTION OF THE DRAWINGS

Figure 1 shows the secondary structures of a hammerhead ribozyme (A; SEQ ID NO:6) and of a maxizyme (B; SEQ ID NOS:7 and 8). The sequences of substrates are shown in SEQ ID NOS:9, 10 and 11.

Figure 2 shows the types of translocation of CML and a problem caused by a ribozyme targeting a BCR-ABL chimeric mRNA.

Figure 3 shows each design of a wild type ribozyme targeting bcl-2 (A; SEQ ID NO:12), an HIV-1 trans-maxizymen (B;SEQ ID NOS:1 and 2), and a CML trans-maxizyme (C; SEQ ID NOS:3 and 4). Regarding trans-maxizymes, the activated structure and inactivated structure thereof are also shown. The sequence of a target site is shown in SEQ ID NOS:13, and the sequences of mRNAs which bind to the sensor sites of ribozymes are shown in SEQ ID NOS:14, 15 and 16.

Figure 4 shows the schematic diagram of a maxizyme which is capable of recognizing an identical intramolecular site of an RNA molecule via its sensor and cleaving another site of the molecule, and the miniaturization of the maxizyme.

Figure 5 shows the allosteric control of a maxizyme which is capable of recognizing an identical intramolecular site of an RNA molecule via its sensor and cleaving another site of the molecule.

Figure 6 (A and B) shows the results of *in vitro* cleavage tests with trans-maxizymes.

Figure 7 shows the secondary structure of $tRNA^{Val}$ T-MzL (A; SEQ ID NO:17), and the results of Northern blotting showing intracellular expression (B) and location (C) of a $tRNA^{val}$-added trans-maxizyme, which is intracellularly expressed. Symbols N and C are nucleus and cytoplasm, respectively.

Figure 8 shows the results of a Tunel test by which apoptosis caused by a HIV-1 trans-maxizyme was examined (A), and the HIV-1 virus infection inhibiting effect of the trans-maxizyme (B).

Figure 9 shows the results of a Tunel test examining apoptosis caused by a CML trans-maxizyme in various cells.

Figure 10 (A to E) shows the results of Western blotting analysis on the activation of bcl-2 and caspase-3 when a trans-maxizyme was expressed in a cultured cell.

DETAILED DESCRIPTION OF THE INVENTION

The present invention is described in detail below, referring to the drawings.

Below, there is described a design of a nucleic acid enzyme (a trans-maxizyme) which, by binding with a particular RNA sequence, expresses a cleavage activity towards another particular target RNA.

Maxizymes, which have been developed by the present inventors up till now, have independently 2 active center regions and 2 substrate-binding regions (Kuwabara, T., Warashina, M., Tanabe, T., Tani, K., Asano, S, and Taira, K. (1998a) A novel allosterically trans-activated ribozyme, the maxizyme, with exceptional specificity in vitro and in vivo. Mol. Cell 2, 617-627) (Figure 1B). Thus, there has been constructed a system which binds to the junction site of an abnormal BCR-ABL chimeric mRNA in a substrate-binding region, while binding to the most efficient cleavage sequence located far from the junction in another substrate-binding region, and cleaves the substrate after the (GUC) triplet (Kuwabara, T. et al., *supra).* That is to say, one substrate-binding region acts as an "eye" recognizing an abnormal substrate, and the other substrate-binding region acts as an arm to perform a "true" function of ribozyme of cleaving the substrate.

In this construction, it was not until binding of either one of the substrate-binding regions to the junction site of an abnormal BCR-ABL chimeric mRNA that a stable dimeric structure capable of expressing ribozyme activity was formed. That is, the stability of a dimer depends on the formation of a base pair in stem II portion. When the stability of this base pair is too high, a dimeric minizyme forms a dimeric structure by itself (although no substrates exist). Regardless of whether one substrate-binding region recognizing a substrate as an "eye" works or not, the other substrate-binding region located on the side of cleaving substrate of a maxizyme binds to a target substrate region. Since a sequence corresponding to a cleaved portion also exists on normal mRNAs such as ABL mRNA and BCR mRNA, non-specific substrate cleavage, which is a problem occurring in conventional hammerhead ribozymes, would occur. However, when the stability of a base pair in stem II portion is too low, formation of an active dimeric structure becomes difficult, and there occurs a similar problem to conventional minizymes having an extremely low substrate-cleavage efficiency (Figure 1A, right).

In the present invention, stability of a base pair in such a delicate stem II portion is achieved to overcome both problems stated above, and a nucleotide sequence forming an active ribozyme only in the presence of abnormal mRNA is devised.

Moreover, in the present invention, where this system is constructed, a side recognizing abnormal mRNA and specifically binding to it does not cleave a substrate. So, the sequence of an active center region, which is necessary for cleavage of an RNA strand and provides a scaffold for capturing a metal ion, can be trimmed, and thereby conventional maxizyme can further be miniaturized (Figure 4, right). The present invention is designed such that an active dimeric structure is formed only when a target mRNA (e.g. BCR-ABL chimeric mRNA) binds to 2 substrate-binding regions in a maxizyme properly, and that merely an inactive structure is formed in the presence of a non-target normal mRNA (e.g. ABL mRNA). When a normal mRNA sequence approaches a substrate-binding region on the junction-recognizing side, an inactive structure is adopted. Differing from an active dimeric structure, the structure of an active center site capturing a magnesium ion essential to cleavage activity of a hammerhead ribozyme is out of balance. As a result, a non-specific cleavage of normal mRNA by the dimeric minizyme does not occur. Furthermore, even though a substrate-binding region on the cleaving side of a substrate, singly binds to the substrate (in this case, substrates to be bound could be both normal mRNA and target mRNA), a structure wherein an active site closes is formed, unless a target BCR-ABL chimeric mRNA sequence approaches in the other substrate-binding region. As a result, since magnesium ion essential to cleavage cannot be captured, cleavage does not occur (Figure 5).
The nucleic acid enzyme, i.e. trans-maxizyme, of the present invention can be employed for the treatment of any disease including cancers, such as solid tumors and blood tumors, and infections such as viral infections, or it can be employed for tissue-specific regulation of gene expression.

(1) Cancers

The trans-maxizymes of the present invention can be used for treatment of solid tumors and blood tumors.

(i) Solid tumors

In solid tumors including, but not limited to, lung cancer, stomach cancer and breast cancer, the expression of a plurality of oncogenes is often amplified. In this case, the trans-maxizyme can recognize mRNAs for two different oncogenes, whose expression is amplified, by the two recognition sequences of the trans-maxizyme, and cleave one mRNA, thereby inhibiting or suppressing an ability of a cancer cell to proliferate it and/or an ability of a cancer cell to metastasize it. Because the trans-maxizyme does not form an activated structure thereof in a normal cell in which the oncogenes are not amplified, no cytotoxic effect, which is an adverse effect, will occur. Examples of diseases and target genes to which the trans-maxizyme of the invention is applicable are as follows.

| Diseases | Target genes |
|---|---|
| Breast cancer | cyclin D, hst-1 |
| Lung cancer | c-myc, c-erbB2 |
| Stomach cancer | c-met, c-erbB2 |

The mRNAs for these oncogenes can be recognized by one recognition sequence of the trans-maxizyme of the invention, while an mRNA for a gene essential for cells, such as a transcriptional factor TBP, is recognized by the other recognition sequence of the trans-maxizyme, resulting in that the expression of the oncogene mRNA is inhibited or suppressed to kill cancer cells alone in which the oncogene is amplified.

(ii) Blood tumors

Most of blood tumor-causing genes are chimeric fusion genes, which are responsible for chromosomal transiocation or the like. The junction site of the chimeric fusion mRNA can be recognized by one recognition sequence of the trans-maxizyme of the invention, while an mRNA for a gene essential for cells, such as a transcriptional factor TBP, is recognized by the other recognition sequence of the trans-maxizyme, resulting in that the expression of the chimeric fusion gene is inhibited or suppressed to kill cancer cells alone in which the chimeric fusion gene is expressed. Examples of diseases and target chimeric fusion genes to which the trans-maxizyme of the invention is applicable are as follows.

| Diseases | Target genes |
|---|---|
| Acute myelocytic leukemia (AML) | PML-RARalpha |
| Osteomyelodysplasia (MDS) | MOZ-CBP |
| B-cell leukemias | MLL-Bcl2 |

(continued)

| Diseases | Target genes |
|---|---|
| T-cell leukemias | TAL1-TCR |

(2) Infections

The trans-maxizymes of the present invention can also be used for treatment of infectious diseases including, but not limited to, AIDS, human T cell leukemia, papilloma and hepatitis, which are caused by viruses such as HIV (human immunodeficiency virus), HTLV (human T cell leukemia virus), HPV (human papilloma virus) and HCV (hepatitis C virus), respectively. For HIV, the above-mentioned description should be referred to.

(i) Human T cell leukemia

The mRNA for Tax, which is a major gene of HTLV, can be recognized by one recognition sequence of the trans-maxizyme of the invention, while an mRNA for a gene essential for cells, such as a transcriptional factor TBP, is recognized by the other recognition sequence of the trans-maxizyme, resulting in that the expression of the Tax mRNA is inhibited or suppressed to kill cells alone which are infected with HTLV.

(ii) Papilloma

The mRNA for E6 or E7, which is a major gene of HPV, can be recognized by one recognition sequence of the trans-maxizyme of the invention, while an mRNA for a gene essential for cells, such as a transcriptional factor TBP, is recognized by the other recognition sequence of the trans-maxizyme, resulting in that the expression of the E6 mRNA or E7 mRNA is inhibited or suppressed to kill cells alone which are infected with HPV

(iii) Hepatitis

The mRNA for NS3, which is a major gene of HCV, can be recognized by one recognition sequence of the trans-maxizyme of the invention, while an mRNA for a gene essential for cells, such as a transcriptional factor TBP, is recognized by the other recognition sequence of the trans-maxizyme, resulting in that the expression of the mRNA for NS3 is inhibited or suppressed to kill cells alone which are infected with HCV

(3) Tissue-specific targeting

In the present invention, it is possible to prepare a trans-maxizyme functioning tissue-specifically. Examples of the tissue include organs, such as liver, stomach, kidney, spleen, intestine, brain, heart, etc., connective tissues such as bone, muscles, and nerve tissues. For example, alpha fetoprotein (AFP) is a protein which is highly expressed specifically in the hepatocyte. If a trans-maxizyme is designed such that the AFP mRNA is recognized by one recognition sequence of the trans-maxizyme of the invention while a target mRNA by the other recognition sequence of the trans-maxizyme, the resulting trans-maxizyme will be able to be employed for tissue-specific regulation of gene expression in the liver.

Thus, for the purpose of designing a ribozyme capable of disrupting a chimeric RNA or mRNA, the present inventors have found a maxizyme, which targets a junction sequence and controls cleavage of normal RNA by the ribozyme, the normal RNA sharing a portion of the chimeric RNA, while not damaging host cells or tissues. Examples of suitable hosts include animals, especially mammals including a human, and plants.

The above-described maxizyme is designed so that both a sequence-recognizing site (a sensor site) acting as an eye and a sequence complementary to a target sequence to be cleaved by the maxizyme exist in an identical molecule (an mRNA). In the present invention, there is also assumed a case where a site recognized by the eye of a maxiyzme and a cleavage active site do not exist in an identical molecule. That is to say, a maxizyme is designed to cleave an mRNA, when it recognizes a different mRNA. The use of the maxizyme of the present invention enables inhibition of expression of other mRNAs, on the condition that a particular mRNA expresses. This suggests that only virus-infected cells can be killed selectively by cleaving an mRNA essential to survival of host cells in the presence of a particular mRNA which is expressed by a virus-infected cell or a virus itself.

EXAMPLES

The present invention is further specifically described in the following examples. The examples are not intended to limit the scope of the invention.

Example 1. Design of trans-maxizyme

Each monomer of the trans-maxizymes shown in Figures 3B and 3C was produced by chemical synthesis of protected RNAs using a DNA/RNA synthesizer (model 394; Applied Biosystems, Division of Perkin Elmer Co. (ABI), Foster City, CA), deprotection, desalting, and purification by PAGE. Both 3'-sides of T-MzL and B-MzL and 5'-sides of T-MzR and B-MzR were designed so that these sides were able to bind complementarily to a target site of bcl-2 mRNA having an apoptosis inhibiting function to form a cleavage active site. 5'-side of T-MzL and 3'-sidess of T-MzR were designed so that these sides were able to recognize an HIV-1 tat mRNA (an HIV-1 trans-maxizyme, T-Mz, Figure 3B). In contrast, 5'-side of B-MzL and 3'-side of B-MzR were designed so that these sides were able to recognize a BCR-ABL mRNA (a CML trans-maxizyme, B-Mz, Figure 3C). Where cells were not infected with HIV-1 viruses and so had no HIV-1 tat mRNA existing therein, as shown on the right side of Figure 3B, host cell bcl-2 mRNA was designed not to be cleaved by the reason that uptake of a metal ion essential to activity becomes impossible by substantial change of a structure including an active center site. Moreover, where a BCR-ABL mRNA, a marker gene of chronic myelocytic leukemia does not exist in cells, as shown in the downside of Figure 3C, in spite of binding to an ABL mRNA comprising a partially identical sequence, host cell bcl-2 mRNA was designed not to be cleaved by the reason that uptake of a metal ion essential to activity becomes impossible by substantial change of a structure including an active center site. As a control, there was prepared a wild type hammerhead ribozyme (Figure 3A, Wt Rz), and inactive trans-maxizymes (I-T-Mz and I-B-Mz) which became incapable of uptake of a metal ion by substituting each of the active center site sequences (CUGAUGA) of both HIV-1 and CML MzRs by the other sequence (CUAAUGA).

In contrast to a conventional maxizyme (Mz) which exerts a sensor function and a cleavage function towards an identical mRNA, the maxizymes designed in the present invention were expected to exert those functions between two mRNA molecules, and so they are named a "trans-maxizyme".

Example 2. *In vitro* cleavage activity and substrate specificity of trans-maxizyme

Cleavage activity and substrate specificity of the trans-maxizymes prepared in Example 1 can be evaluated as follows. Using T4 polynucleotide kinase (Takara Shuzo, Kyoto, Japan), a partial sequence (300 mer) of bcl-2 mRNA was labeled with [$\gamma$-$^{32}$P]-ATP. Each enzyme (1$\mu$M) was incubated with 2nM bcl-2 mRNA, the 5'-terminus of which was labeled with $^{32}$P. The resulting product and trans-maxizyme were mixed in a Tris-HCl buffer (pH 8.0). Reaction was initiated by addition of 10mM $MgCl_2$ to the buffer containing each enzyme and a substrate, and the reaction was performed at 37°C for 60 minutes. Then, the reaction mixture was subjected to an electrophoresis using 5% polyacrylamide/7M urea gel. Finally, existence or nonexistence of cleavage was detected by a BAS 2000 image analyzer.

Each experimental result for HIV-1 and CML is shown in Figures 6A and 6B, respectively. Regarding an HIV-1 trans-maxizyme, it was confirmed that the trans-maxizyme cleaved specifically a bcl-2 mRNA in the presence of HIV-1 tat mRNA (T-Mz). Regarding a CML trans-maxizyme, it was confirmed that the trans-maxizyme did not cleave a bcl-2 mRNA in the presence of a normal ABL mRNA, and specifically cleaved it in the presence of a BCR-ABL mRNA (B-Mz). Assay regarding activity of the trans-maxizymes was performed by incubation under conditions of enzyme saturation (one metabolic turnover), at 37°C for 60 minutes, using 10mM $MgCl_2$ and 50mM Tris-HCl (pH 8.0).

Moreover, in both cases, regardless of the presence or absence of a disease-specific mRNA, the wild type ribozyme Wt Rz (Figure 3A) cleaved a bcl-2 mRNA. Furthermore, I-T-Mz and I-B-Mz whose metal ion uptake activity was inactivated, and monomeric components of the trans-maxizymes (T-MzL, T-MzR, B-MzL, B-MzR), can not cleave mRNA by themselves.

Example 3. Application of trans-maxizyme to gene therapy

There are two types of methods for expressing a ribozyme *in vivo*: a method in which a synthetic ribozyme is encapsulated with a cationic lipid membrane or the like and introduced into a cell of an organism (Malone, RW. Felgner, PL., Vermn, IM (1989) Proc. Natl. Acad, Sci., USA 86, 6077) and a method in which a ribozyme is introduced as vector DNA (using a viral vector etc.) and expressed in a cell of an organism (Friedmann. T., Roblin. R. (1972) Science 175, 949).

An expression vector comprising DNA encoding the trans-maxizyme of the present invention can be prepared by incorporating a ligated form (e.g. $tRNA^{val}$-MzL, $tRNA^{val}$-MzR) obtained by ligating an RNA polymerase promoter sequence such as a pol II promoter or a pol III promoter (e.g. tRNA, snRNA), a terminator sequence and a maxizyme sequence, into a vector such as pUC19 (Takara), pGREEN LANTERN (Lifetech Oriental) or pHaMDR (HUMAN GENE THERAPY 6:905-915 (July 1995)). As an example, secondary structure of $tRNA^{val}$T-MzL to be experssed is shown in Figure 7A.

The thus prepared expression vector can be introduced into a cell as follows:

(1) Lipofection method

A cell surface is negatively charged. Thus, a complex is produced with a vector of interest (a DNA double-stranded cyclic plasmid) and a cationic lipid (a lipofection reagent (e.g. lipofectin) and introduced into a cell.

(2) Viral vector method

This method has a higher efficiency than the method (1) above. A vector is prepared by selecting only a part necessary for gene expression from among gene information of a virus and incorporating therein a sequence having a therapeutic effect (DNA sequence of $tRNA^{val}$ MzL or $tRNA^{val}$ MzR). Then, this vector is integrated into DNA of a cell of interest by the capability of the virus.

A promoter sequence of RNA polymerase III is added to a maxizyme in a vector sequence (e.g. Figure 7A). A trans-maxizyme is expressed at a high expression level by transcribing an RNA sequence having a therapeutic effect from a vector DNA introduced into a cell by means of an action of RNA polymerase III, which originally functions in a cell.

The results obtained by examining both expression of a HIV-1 trans-maxizyme in an NP2/CD4/CXCR4 cell (acquired from the Institute of Medical Science, the University of Tokyo) and expression of a CML trans-maxizyme in a BV173 cell by Northern hybridization are shown in Figure 7B. An RNA extracted from a cell was subjected to agarose gel electrophoresis to transfer it onto a nucleic acid adsorption membrane, and then a DNA probe having a sequence complementary to a trans-maxizyme whose 5'-teminus was labeled, was hybridized therewith. After that, the presence or absence of expression and localization was detected by a BAS2000 image analyzer. As a result, it was found that both trans-maxizymes expressed at high level, and were transported to and located in cytoplasm via expression dependent on a tRNA promoter used. Furthermore, by examining also expression of bcl-2, Tat, ABL and BCR-ABL mRNA, it was found that each of these was located mainly in cytoplasm (Figure 7C). In the figure, C represents a cytoplasm and N represents the nucleus. That a trans-maxizyme, a target mRNA and a disease-specific mRNA show a similar localization is important for exerting an effect in a cell.

Example 4. Viral infection inhibiting effect of trans-maxizyme

Viral infection inhibiting effect of the HIV-1 trans-maxizyme was analyzed. When the trans-maxizyme can effectively cleave a bcl-2 mRNA, expression of the bcl-2 protein is inhibited and cells are led to apoptosis. Thus, whether apoptosis occurred or not was examined by Tunel method. Generally, while apoptosis is in progress, DNA is decomposed. In Tunel method, using TdT (terminal deoxynucleotidyl transferase), the thus obtained DNA fragment was bound with FITC-labeled dUTP. When the obtained product was observed with a fluorescent microscope, cells in which apoptosis occurs emit fluorescence. This experiment was carried out using In situ cell death detection kit, Fluorescein (Roche Diagnostics).

As shown in Figure 8A, cells into which HIV-1 trans-maxizymes (T-Mz) were introduced emitted fluorescence and apoptosis at a significantly advanced stage was observed. In cells wherein an HIV-1 tat mRNA did not express (shown as (-) tat), however, apoptosis did not occur even after T-Mz was introduced, and this showed a high sensor ability. Although some extent of apoptosis was observed with Wt Rz, it was clearly shown by observation of cell image that the progressive rate of apoptosis was significantly low. Furthermore, no apoptosis was observed using monomers (T-MzL, T-MzR) and an inactive trans-maxizyme (T-I-Mz).

Subsequently, the extent to which a trans-maxizyme inhibits actual HIV-1 virus infection was examined. 30,000 cells were placed into a 24 well plate, and trans-maxizyme (T-Mz) was introduced therein on the following day. Further on the following day (at 24 hours after transfection), 10ng/mL HIV (at a concentration of p24) was infected therewith. After that, an assay was performed every 2 days (D2, D4 and D6; Figure 8B right). As an assay method, a fluorescent antibody method was adopted, wherein a serum from an HIV patient was used as a primary antibody. Percentage (%) of HIV antigen positive cells was obtained, and the average value of 2 measurements is shown in Figure 8B. A wild type ribozyme (Wt Rz, Figure 3A) inhibited a certain extent of viral infection on the $4^{th}$ day, but after the HIV-1 virus infection burst out progressing on the $6^{th}$ day, the inhibiting ability of the wild type ribozyme was significantly reduced (see a blue bar in the middle of D6). A trans-maxizyme constantly showed an extremely high effect and killed all the virus-infected cells, and therefore HIV antigen positive cells became nearly 0% (see a red bar on the right of D6).

Example 5. Cancer cell killing effect of trans-maxizyme

Subsequently, cancer cell killing effect of a CML (chronic myelocytic leukemia) trans-maxizyme was analyzed. Principles of Tunel method and an experimental method used herein are the same as stated above, with only the exception being the variety of cells used herein. As cells which do not express a BCR-ABL mRNA, a CML specific mRNA, there were herein used H9 cells, HL60 cells, BV173 cells as human CML cells, Primary CML cells, and BaF3/p210$^{BCR-ABL}$ cells obtained by expressing a human BCR-ABL mRNA in BaF3 cells established from a mouse. After immobilizing cells and uniformly staining DNA, Propidium Iodide (PI) was added thereto to allow all the cells to emit on the low wavelength side (red color). Degree of apoptosis can be shown by the number of apoptosis cells represented by green color fluorescence, in the total cells represented by red color fluorescence.

As shown in Figure 9, T-Mz caused a high frequency of apoptosis only to BV173 cells as human CML cells, and Primary CML cells. Although a BCR-ABL mRNA was expressed in BaF3/p210$^{BCR-ABL}$ cells, apoptosis did not occur because a bcl-2 mRNA having a mouse-derive sequence was expressed. Wt Rz did not cause apoptosis since it did not have a target mRNA sequence just as with BaF3/p210$^{BCR-ABL}$ cells. However, Wt Rz caused apoptosis in cells other than the above cells regardless of whether a BCR-ABL mRNA expressed or not.

Example 6. Target mRNA cleaving effect of trans-maxizyme in cell

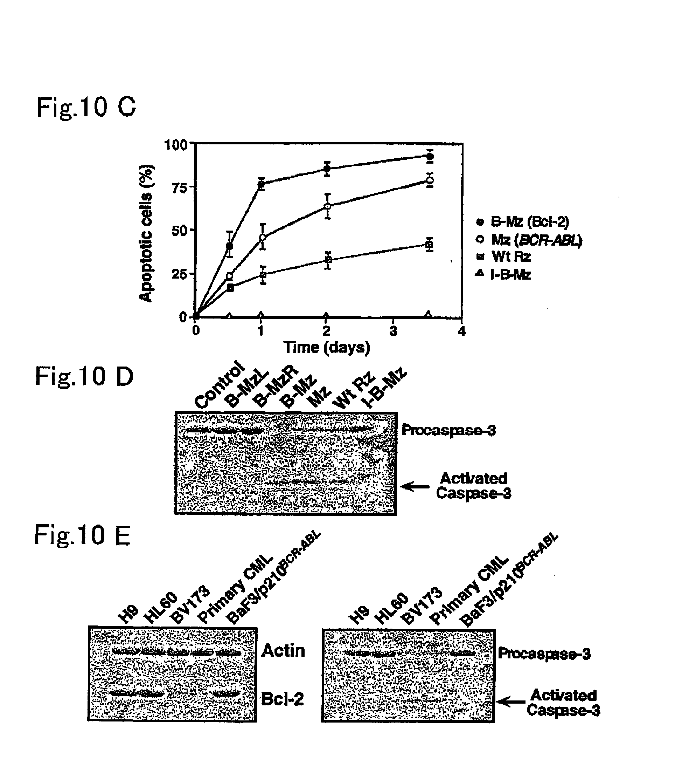

Subsequently, target mRNA cleaving effect of trans-maxizymes was examined. For apoptosis signal transmission and achievement of apoptosis, coordinated actions of aspartic acid-specific cysteine protease (known as caspase) are needed. Figure 10A shows results of Western blotting performed in respect of a bcl-2 protein expression inhibiting effect of the HIV-1 trans-maxizyme, and Figure 10B shows results of Western blotting performed in respect of activation of Caspase-3, which is a caspase activated at the end of apoptosis cascade. A cell extract was subjected to SDS-PAGE with 15% polyacrylamide. Using a rabbit polyclonal antibody $\alpha$ CPP32 recognizing both procaspase-3 and processed p17 (caspase-3) (this antibody was kindly provided by Prof. Hong-Gang Wang, College of Medicine, University of South Florida), activation of procaspase-3 was detected. Blocking and detection were carried out according to the method of Dubrez *et al.* (Dubrez et al., Blood, 7, 2415-2422, 1998) With regard to bcl-2, it was detected using a rabbit polyclonal antibody (Cosmo Bio Co., Ltd.) against bcl-2.

As shown in Figure 10A,Wt Rz inhibited expression of bcl to a small extent, regardless of the presence or absence of a tat mRNA. In contrast, in the use of a trans-maxizyme, it was observed that expression level of bcl-2 did not change when a tat mRNA did not exist, and that the expression was inhibited only when a tat mRNA existed. When other maxizyme monomers (MzL, MzR) or inactivated maxizymes were used, reduction of bcl-2 expression was not observed.

Moreover, with regard to inhibition of bcl-2 expression, reduction of procaspase-3 and activation of caspase-3 associated with induction of apoptosis, were set as indicators. In the use of Wt Rz, caspase-3 was activated regardless of the presence or absence of a tat mRNA. In contrast, in the use of a trans-maxizyme, activation of caspase-3 was confirmed only where a tat mRNA existed.

When other maxizyme monomers (MzL, MzR) or inactivated maxizymes were used, activation of caspase-3 was not confirmed even where a tat mRNA existed (Figure 10B).

Figure 10C is a graph showing a comparison regarding apoptotic effects between a CML trans-maxizyme (B-Mz) and a previously constructed maxizyme (Mz) cleaving a BCR-ABL mRNA. The figure shows that a newly constructed trans-maxizyme recognizing a BCR-ABL mRNA and cleaving a bcl-2 mRNA which is essential to cells (that is, actually inhibiting apoptosis more strongly) has a better apoptotic effect than a previously constructed maxizyme recognizing a BCR-ABL mRNA and cleaving it. Figure 10D shows results of Western blotting performed in respect of a bcl-2 protein expression inhibiting effect of a CML trans-maxizyme, and Figure 10E shows results of Western blotting performed in respect of activation of Caspase-3. These figures shows a high intracellular activity and an extremely high specificity of a CML trans-maxizyme, just as with a HIV- 1 trans-maxizyme.

Effect of the Invention

A trans-maxizyme, the nucleic enzyme of the present invention, enables specific inhibition of growth of virus-infected cells and cancerated cells, and induction of apoptosis to those cells with no effects on mRNA of normal cells, and is thereby useful for effective and safe treatment for various diseases. Therefore, the present invention can be used as a pharmaceutical agent, in particular, a pharmaceutical agent providing a therapeutic effect by specifically inducing apoptosis to cancer cells and virus infected cells etc.

## Claims

1. A nucleic acid enzyme having a sensor site and a cleavage active site, wherein the nucleic acid enzyme exhibits a cleavage activity towards a target RNA only when the target RNA binds to the cleavage active site while an RNA, DNA or protein dilTerent from the target RNA binds to the sensor site.

2. The nucleic acid enzyme according to claim 1, having a dimeric structure which is formed from an RNA molecule comprising nucleotide sequence (10) below, and an RNA molecule comprising nucleotide sequence (20) below:

$$5'X^1_l \cdots X^1_h Y^1_l \cdots Y^1_i Z^1_l \cdots Z^1_j 3' \quad (10);$$

and

$$5'Z^2_l \cdots Z^2_n\, Y^2_l \cdots Y^2_m\, X^2_l \cdots X^2_k\, 3' \quad (20),$$

wherein $X^1_l$ to $X^1_h$, $X^2_l$ to $X^2_k$, $Y^1_1$ to $Y^1_i$, $Y^2_l$ to $Y^2_m$, $Z^1_l$ to $Z^1_j$ and $Z^2_l$ to $Z^2_n$ are each independently any one of A, U, T, C or G,

h and k are each an integer of 1 or greater,
i and m are each an integer of 1 or greater,
j is an integer of 1 or greater,
n is an integer of 1 or greater,
$X^1_l \cdots X^1_h$ and $X^2_l \cdots X^2_k$ are nucleotide sequences capable of forming a sensor site,
$Y^1_l \cdots Y^1_i$ and $Y^2_l \cdots Y^2_m$ are nucleotide sequences capable of forming a stem, comprising regions which are able to form a cavity to stably capture a $Mg^{2+}$ ion only when said RNA, DNA or protein binds to a sensor site, and
$Z^1_l \cdots Z^1_j$ and $Z^2_l \cdots Z^2_n$ are nucleotide sequences comprising regions complementary to target RNA sequences adjacent to a target RNA cleavage site.

**3.** The nucleic acid enzyme according to claim 1, wherein the target RNA is an mRNA encoding a protein essential for the survival of a cell.

**4.** The nucleic acid enzyme according to claim 1, wherein the target RNA is an mRNA encoding a protein which prevents apoptosis, such as bcl-2 protein.

**5.** The nucleic acid enzyme according to claim 1, wherein the target RNA is a viral RNA, or an mRNA encoding a protein associated with a disease such as cancer.

**6.** The nucleic acid enzyme according to claim 1, wherein the RNA molecule that binds to the sensor site is an mRNA encoding a disease-associated protein.

**7.** The nucleic acid molecule according to claim 1, wherein the RNA molecule that binds with the sensor site is a tissue-specifically or time-specifically expressing mRNA, or an RNA artificially introduced for the purpose of regulating a nucleic acid enzyme activity.

**8.** The nucleic acid enzyme according to claim 5 or 6, wherein the mRNA encoding a disease-associated protein is an mRNA derived from a oncocyte marker, or virus-derived RNA such as HIV tat mRNA.

**9.** The nucleic acid enzyme according to claim 1, having a dimeric structure which is formed from an RNA molecule comprising nucleotide sequence (1) below and an RNA molecule comprising nucleotide sequence (2) below :

5' GGUCCUGGCC UGAUGAGAGU GAUGAGCUCU UC 3' (1) (SEQ ID NO:1);

and

5' GUCUGACUGU UCAUCGAAAC CGGGUCC 3' (2) (SEQ ID NO:2),

wherein nucleotide Nos. 1 to 9 of nucleotide sequence (1) and nucleotide Nos. 19 to 27 of nucleotide sequence (2) may or may not be modified such that they are complementary to a target RNA sequence adjacent to a target RNA cleavage site, and wherein nucleotide Nos. 20 to 32 of nucleotide sequence (1) and nucleotide Nos. 1 to 12 of nucleotide sequence (2) may or may not be modified such that they are complementary to a sequence within another mRNA specific to an AIDS-causing virus, HIV-1, or to a sequence of another site within tat mRNA of HIV-1.

**10.** The nucleic acid enzyme according to claim 5 or 6, wherein the mRNA encoding a disease-associated protein is a chronic myelocytic leukemia-derived BCR-ABL mRNA.

**11.** The nucleic acid enzyme according to claim 1, having a dimeric structure formed by an RNA molecule comprising nucleotide sequence (3) below and an RNA molecule comprising nucleotide sequence (4) below:

5' GGUCCUGGCC UGAUGAGAGU UAUUGAUGGU CAG 3' (3) (SEQ ID NO:3);

and

5' GAAGGGCUUC UUUCAUCGAA ACCGGGUCC 3' (4) (SEQ ID NO:4),

wherein nucleotide Nos. 1 to 9 of nucleotide sequence (3) and nucleotide numbers 20 to 29 of nucleotide sequence (4) may or may not be modified such that they are complementary to a target RNA sequence adjacent to a target RNA cleavage site, and wherein nucleotide Nos. 20 to 33 of nucleotide sequence (3) and nucleotide Nos. 1 to 14 of nucleotide sequence (4) may or may not be modified such that they are complimentary to a sequence within another mRNA specific to chronic myelocytic leukemia or a sequence of another site within BCR-ABL mRNA.

**12.** The nucleic acid enzyme according to claim 1, wherein a linker sequence, a $tRNA^{Val}$ promoter sequence, or both, is added upstream of the nucleic acid enzyme sequence.

**13.** The nucleic acid enzyme according to claim 1, wherein a pol II promoter sequence or pol III promoter sequence is added upstream of the nucleic acid enzyme sequence.

**14.** The nucleic acid enzyme according to claim 12, which comprises nucleotide sequence (5) or nucleotide sequence (6) below:

5' AAA 3' (5);

and

5' UUU 3' (6).

**15.** The nucleic acid enzyme according to claim 12, wherein the $tRNA^{Val}$ promoter sequence comprises nucleotide sequence (7) below:

5' ACCGUUGGUU UCCGUAGUGU AGUGGUUAUC ACGUUCGCCU AACACGCGAA AGGUCCCCGG UUCGAAACCG GGCACUACAA AAACCAAC 3' (7) (SEQ ID NO:5).

**16.** The nucleic acid enzyme according to claim 1, wherein a delivery agent is attached to the nucleic acid enzyme sequence

**17.** The nucleic acid enzyme according to claim 16, wherein the delivery agent is selected from the group consisting of peptides, peptide mimics, virus-derived proteins, cholesterols, steroids, cholesterol derivatives, fats, vitamins, biotin, folic acid, retinoic acid, proteins, ferritin, LDL, insulin, antibodies, saccharides or oligosaccharides, polyethylene glycol, or homopolymers or copolymers of amino acids.

**18.** The nucleic acid enzyme according to claim 1, wherein an additional sequence, terminator sequence, or both, is added downstream of the nucleic acid enzyme sequence.

**19.** The nucleic acid enzyme according to claim 18, wherein the additional sequence comprises any one of nucleotide sequences (8) to (10) below:

5' AAA 3' (8);

5' AACCGUA 3' (9);

and

5' UUUUU 3' (10).

**20.** An expression vector comprising DNA encoding the nucleic acid enzyme according to claim 1.

**21.** A method of producing a nucleic acid enzyme, wherein the method comprises transcribing into RNA using, as a template, an expression vector DNA comprising DNA encoding the nucleic acid enzyme according to claim 1.

**22.** A gene transfer vehicle comprising the nucleic acid enzyme according to claim 1.

**23.** The gene transfer vehicle according to claim 22, which is in the form of a positively charged liposome.

**24.** A pharmaceutical composition comprising, as an active ingredient, the nucleic acid enzyme according to claim 1, the expression vector according to claim 20, or the vehicle of claim 22.

**25.** The pharmaceutical composition according to claim 24, for use in prevention and/or treatment of a disease caused by a target DNA.

**26.** The pharmaceutical composition according to claim 24 for controlling or inhibiting expression of disease-causing abnormal mENA by allowing the nucleic acid enzyme according to claim 1 to express within an organism.

**27.** A method of specifically cleaving a target RNA using the nucleic acid enzyme according to claim 1.

**28.** The method according to claim 27, wherein the target RNA is an abnormal mRNA which causes a disease.

**29.** A host cell comprising the nucleic acid enzyme according to claim 1, the host cell being selected from the group consisting of prokaryote cells such as *E. coli,* and eukaryotic cells such as human cells, mammalian cells, plant cells and yeast cells.

**30.** A diagnostic agent comprising the nucleic acid enzyme according to claim 1.

Fig.1 A

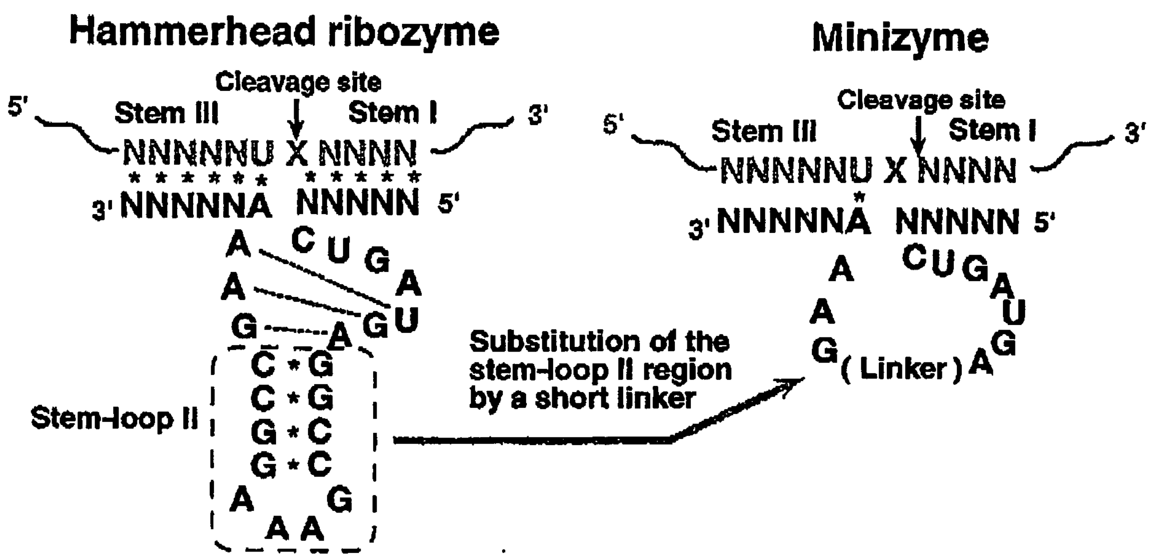
Hammerhead ribozyme
Cleavage site
5'
Stem III
Stem I
3'
NNNNNU X NNNN
3' NNNNNA NNNNN 5'
A C U G A
A
G A G U
Stem-loop II
C G
C G
G C
G C
A G
A A
Substitution of the stem-loop II region by a short linker
Minizyme
Cleavage site
5'
Stem III
Stem I
3'
NNNNNU X NNNN
3' NNNNNA NNNNN 5'
A CUGA
A U
G ( Linker ) A G

Fig.1 B

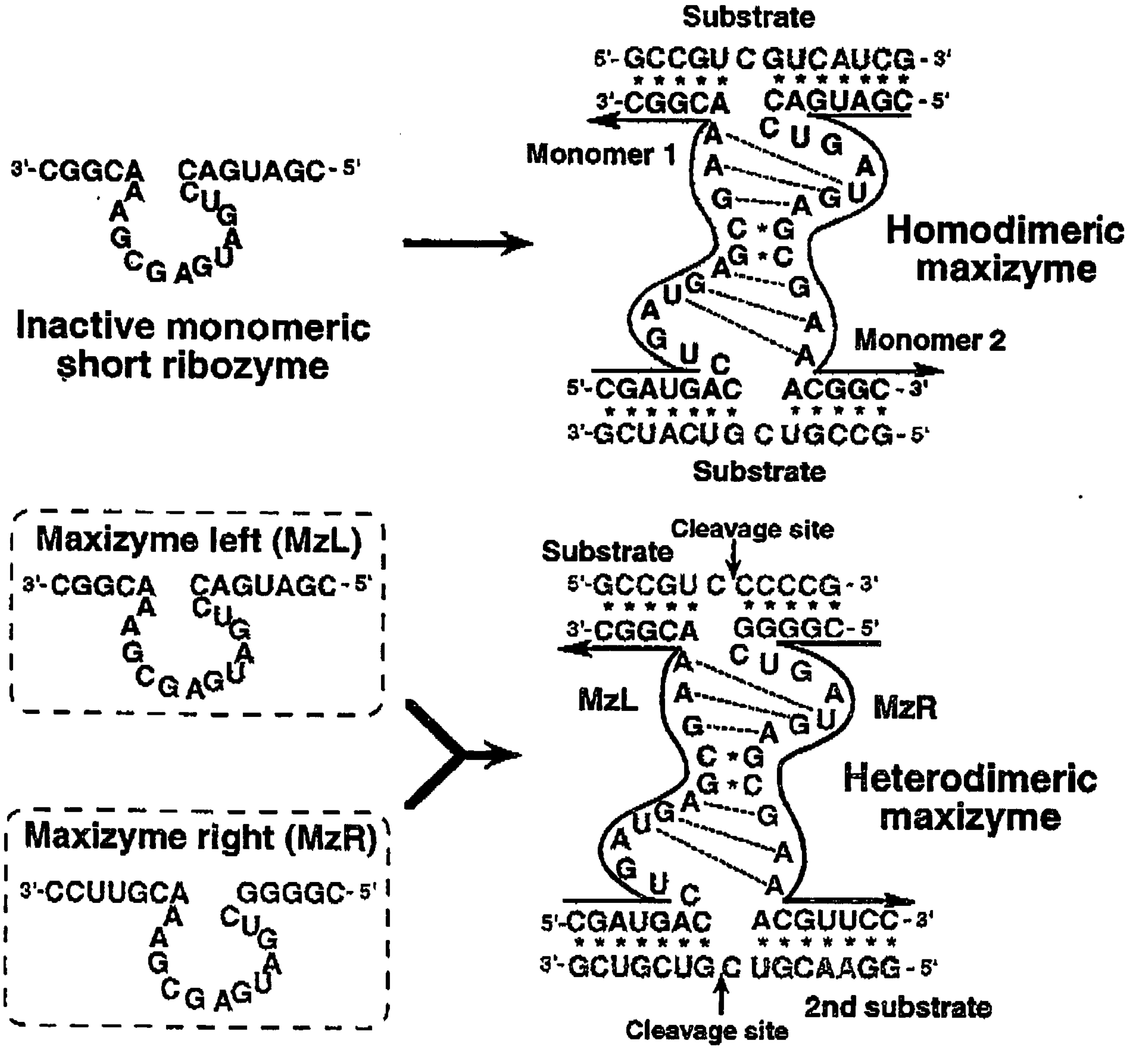
3'-CGGCA CAGUAGC-5'
Inactive monomeric short ribozyme
Substrate
5'-GCCGU C GUCAUCG-3'
3'-CGGCA CAGUAGC-5'
Monomer 1
Homodimeric maxizyme
Monomer 2
5'-CGAUGAC ACGGC-3'
3'-GCUACUG C UGCCG-5'
Substrate
Maxizyme left (MzL)
3'-CGGCA CAGUAGC-5'
Maxizyme right (MzR)
3'-CCUUGCA GGGGC-5'
Substrate
Cleavage site
5'-GCCGU C CCCCG-3'
3'-CGGCA GGGGC-5'
MzL
MzR
Heterodimeric maxizyme
5'-CGAUGAC ACGUUCC-3'
3'-GCUGCUG C UGCAAGG-5'
2nd substrate
Cleavage site

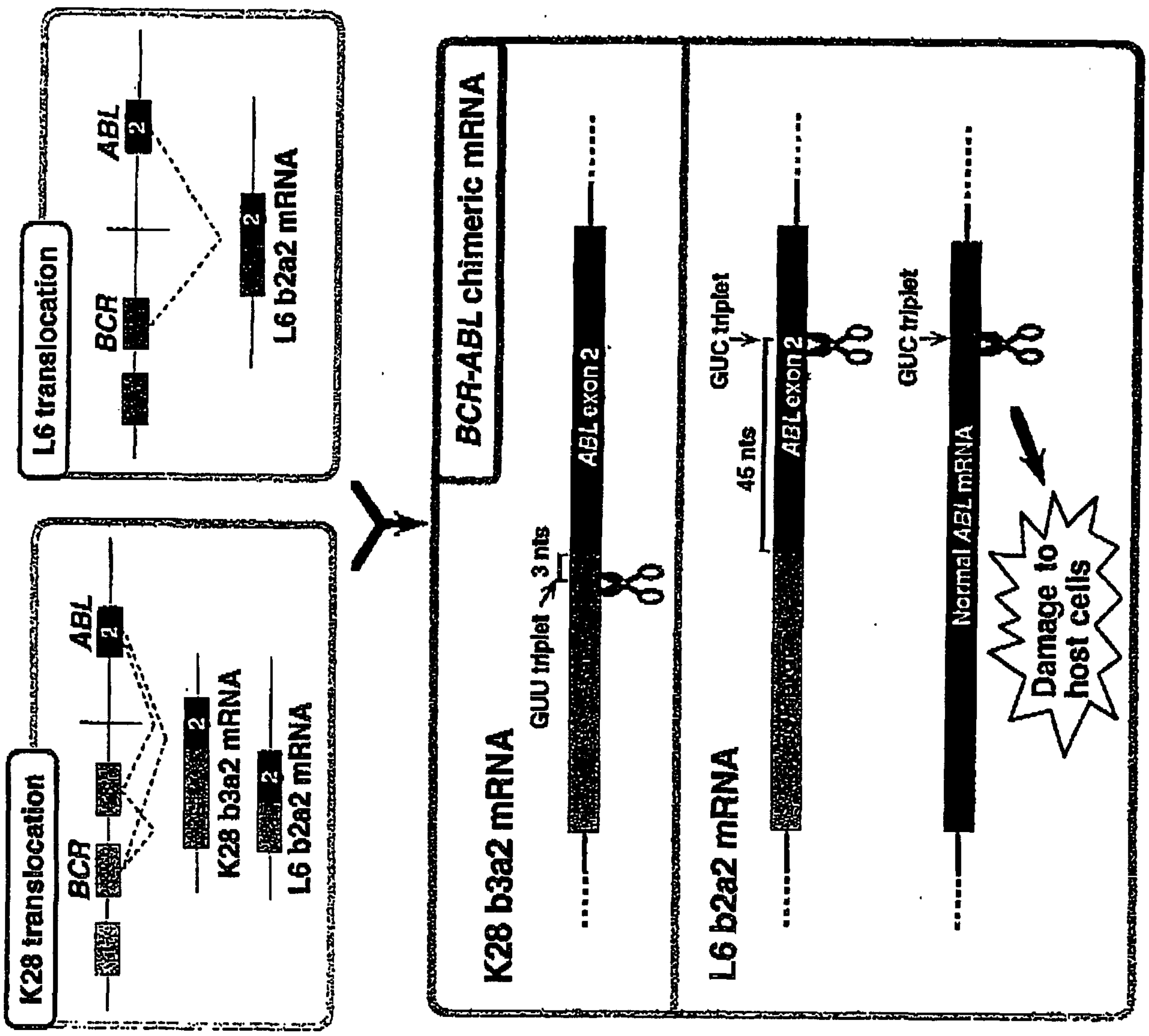
K28 translocation
BCR
ABL
2
K28 b3a2 mRNA
L6 b2a2 mRNA
L6 translocation
BCR
ABL
2
L6 b2a2 mRNA
BCR-ABL chimeric mRNA
K28 b3a2 mRNA
GUU triplet
3 nts
ABL exon 2
L6 b2a2 mRNA
GUC triplet
45 nts
ABL exon 2
GUC triplet
Normal ABL mRNA
Damage to host cells

Fig.2

Fig.3 A

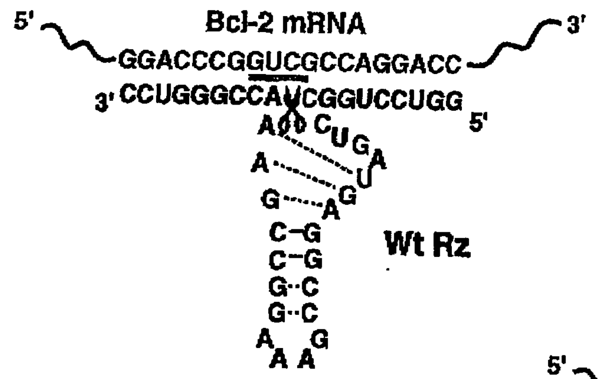
Bcl-2 mRNA
GGACCCGGUCGCCAGGACC
3' CCUGGGCCAUCGGUCCUGG 5'
Wt Rz

Fig.3 B

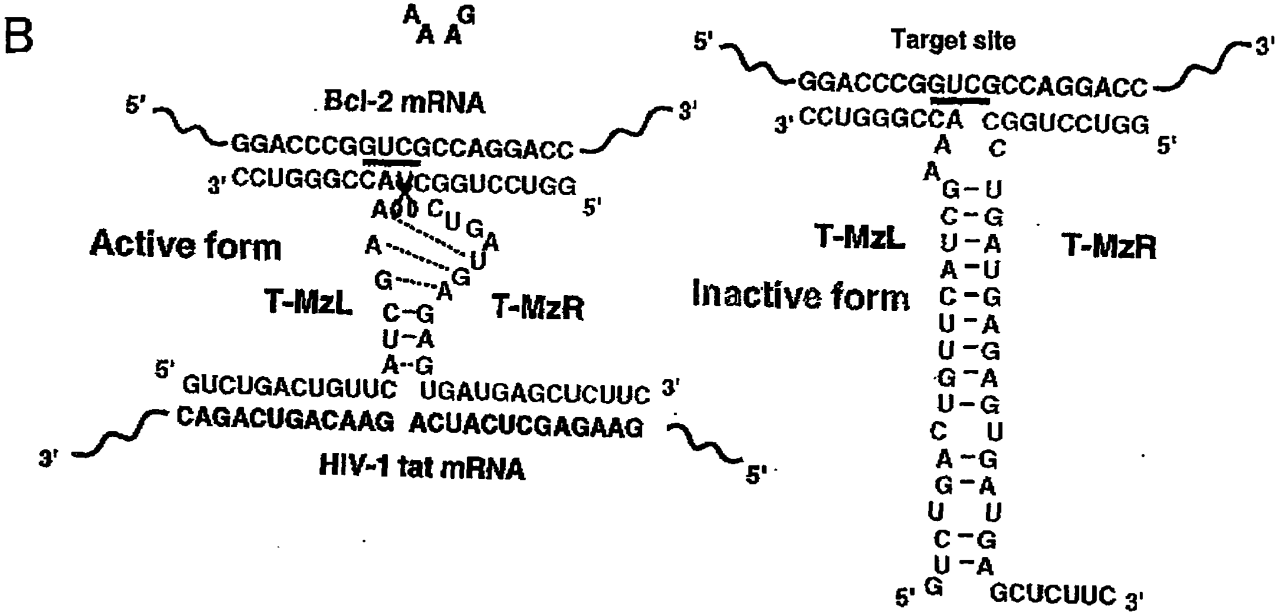
Bcl-2 mRNA
GGACCCGGUCGCCAGGACC
3' CCUGGGCCAUCGGUCCUGG 5'
Active form
T-MzL
T-MzR
5' GUCUGACUGUUC UGAUGAGCUCUUC 3'
CAGACUGACAAG ACUACUCGAGAAG
HIV-1 tat mRNA
Target site
GGACCCGGUCGCCAGGACC
3' CCUGGGCCA CGGUCCUGG 5'
T-MzL
T-MzR
Inactive form
5' G GCUCUUC 3'

Fig.3 C

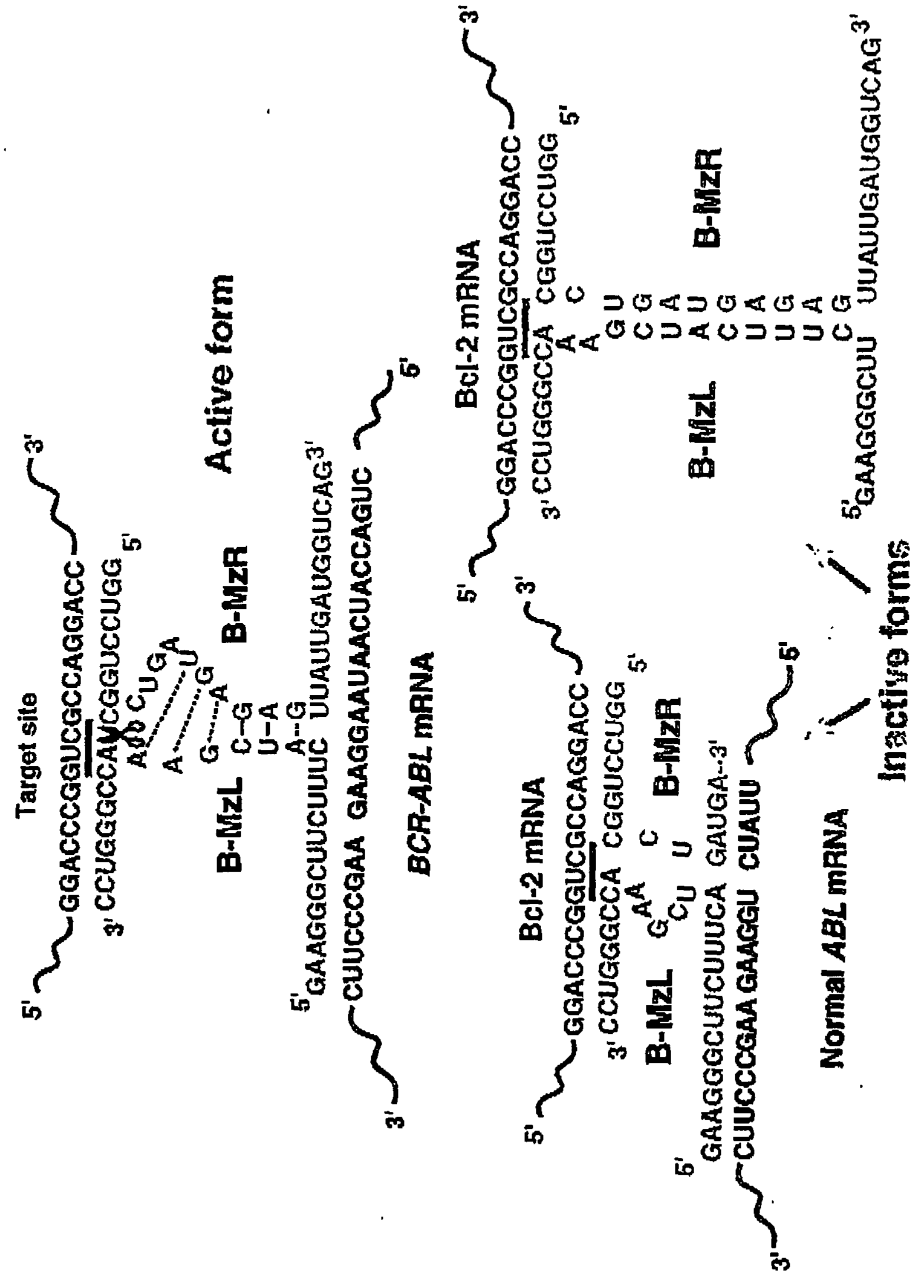

Target site
GGACCCGGUCGCCAGGACC
CCUGGGCCA CGGUCCUGG
B-MzL
B-MzR
GAAGGGCUUCUUUC UUAUUGAUGGUCAG
CUUCCCGAA GAAGGAAUAACUACCAGUC
BCR-ABL mRNA
Active form
Bcl-2 mRNA
B-MzL
B-MzR
Normal ABL mRNA
Bcl-2 mRNA
B-MzL
B-MzR
Inactive forms

Fig.4

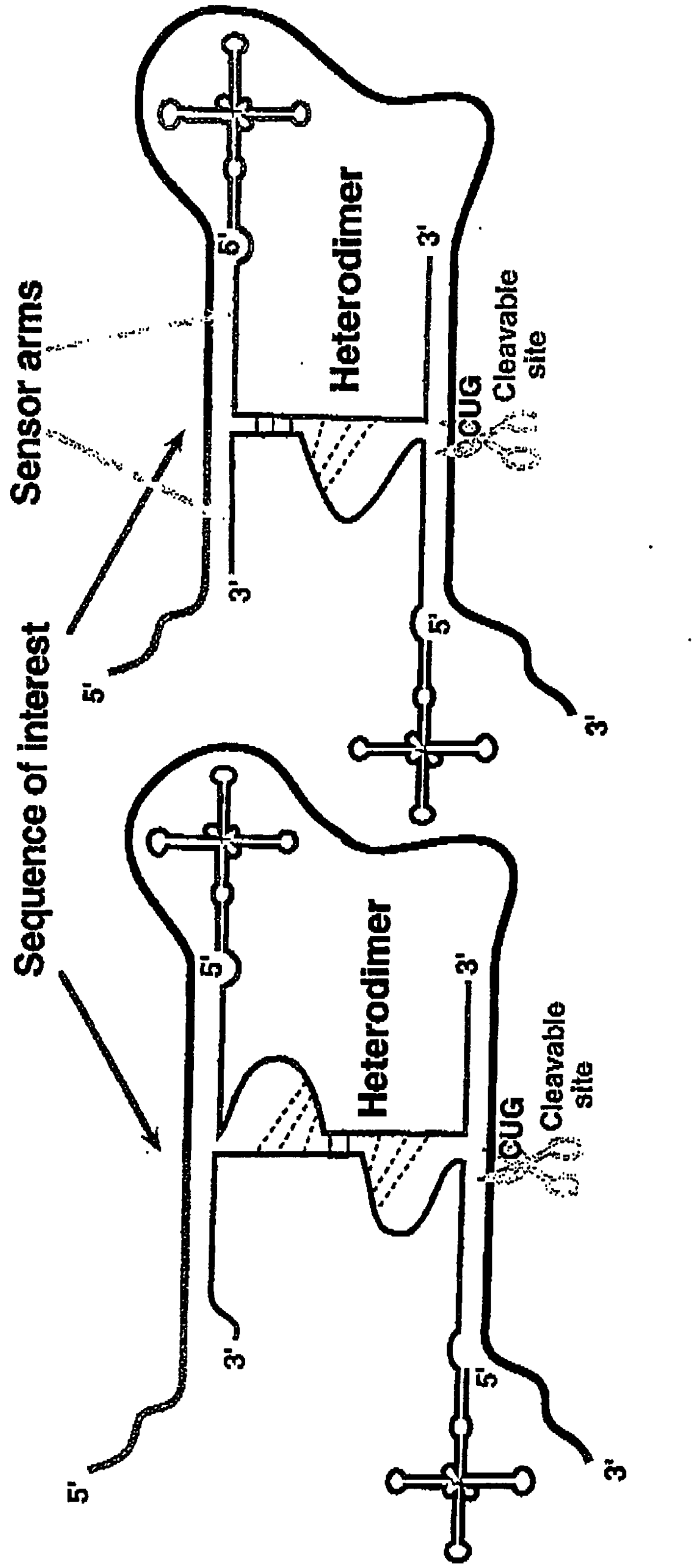
Sequence of interest
Sensor arms
Heterodimer
Heterodimer
5'
3'
5'
3'
CUG
Cleavable site
CUG
Cleavable site

Fig.5

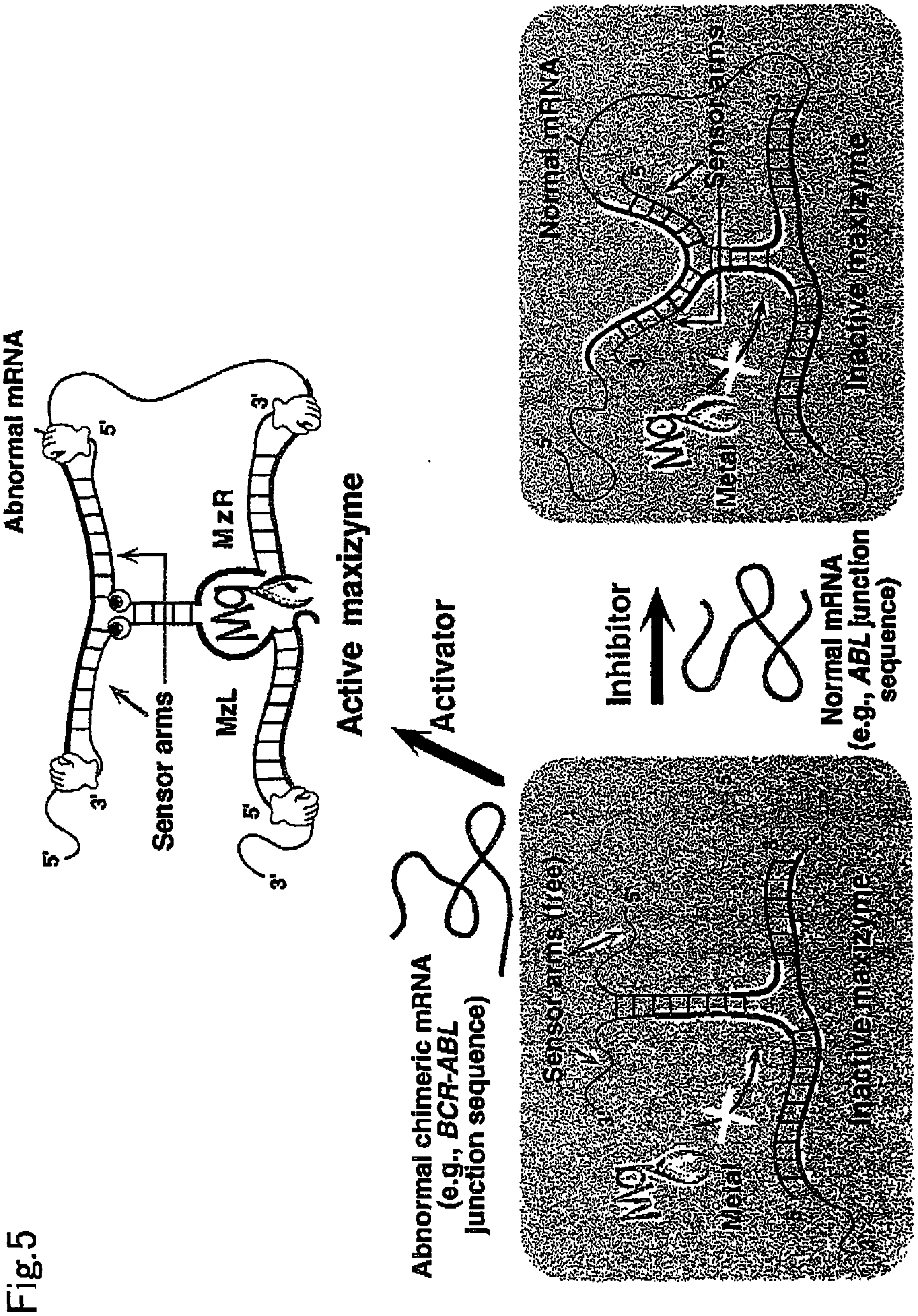

Abnormal mRNA
5'
3'
Sensor arms
MzL
MzR
Active maxizyme
Activator
Inhibitor
Abnormal chimeric mRNA
(e.g., BCR-ABL
junction sequence)
Normal mRNA
(e.g., ABL junction
sequence)
Sensor arms (free)
Metal
Inactive maxizyme
Normal mRNA
Sensor arms
Metal
Inactive maxizyme

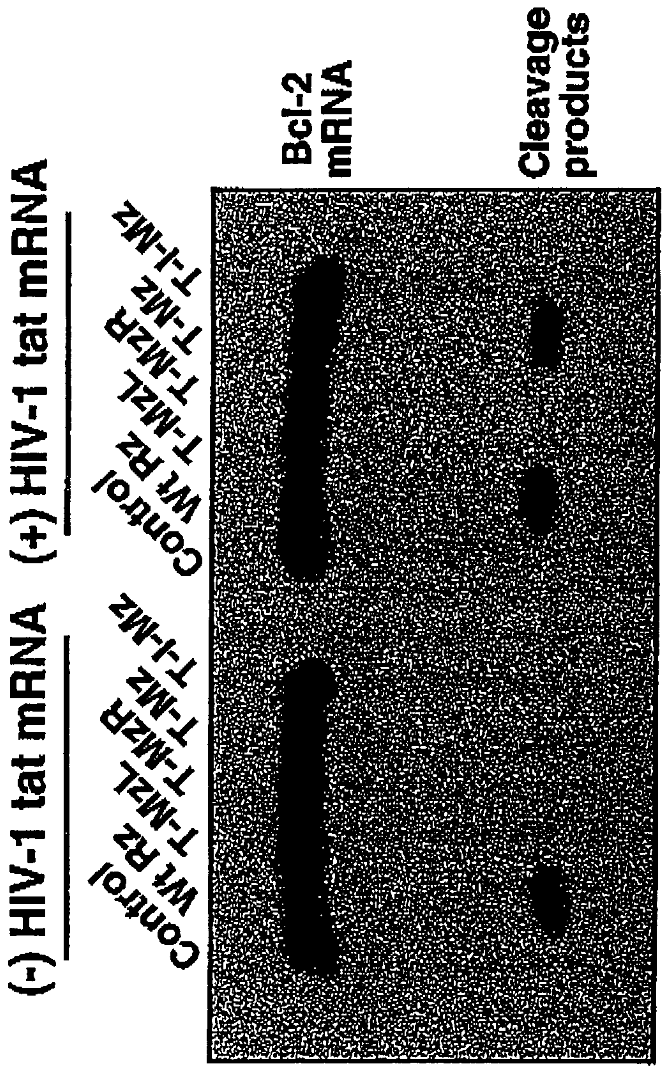
(-) HIV-1 tat mRNA
(+) HIV-1 tat mRNA
Control
Wt Rz
T-MzL
T-MzR
T-Mz
T-I-Mz
Bcl-2
mRNA
Cleavage
products

Fig.6 A

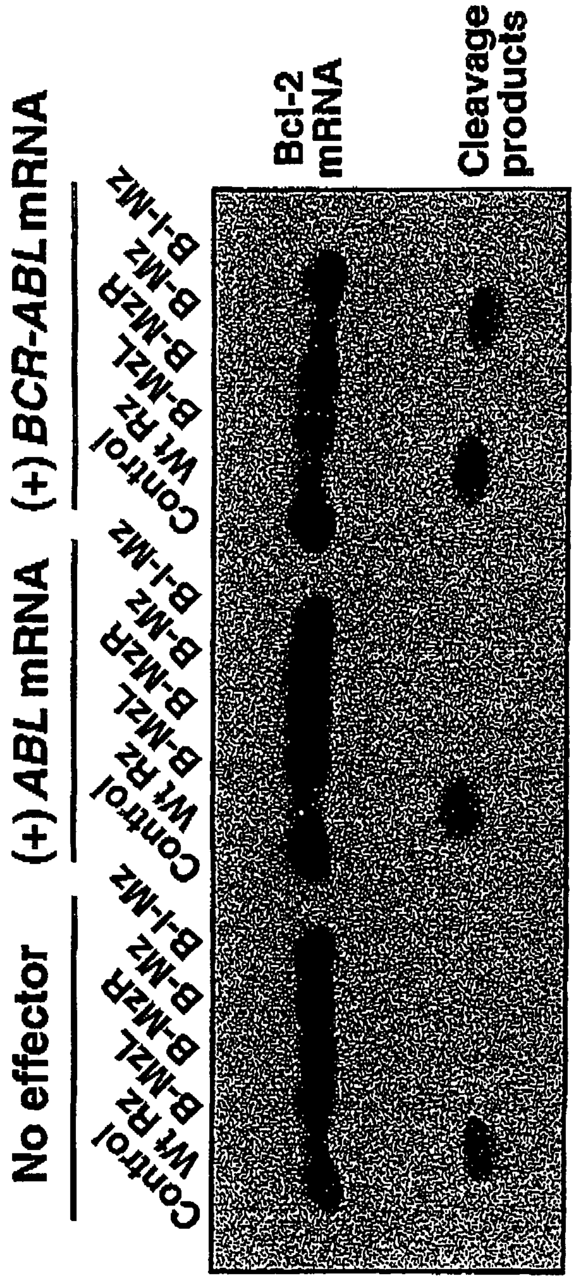
No effector
(+) ABL mRNA
(+) BCR-ABL mRNA
Control
Wt Rz
B-MzL
B-MzR
B-Mz
B-I-Mz
Control
Wt Rz
B-MzL
B-MzR
B-Mz
B-I-Mz
Control
Wt Rz
B-MzL
B-MzR
B-Mz
B-I-Mz
Bcl-2 mRNA
Cleavage products

Fig.6 B

Fig.7 B

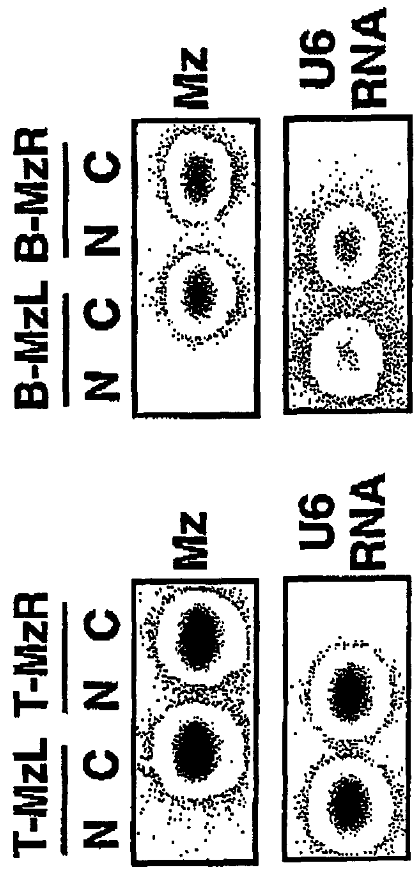


Fig.7 A

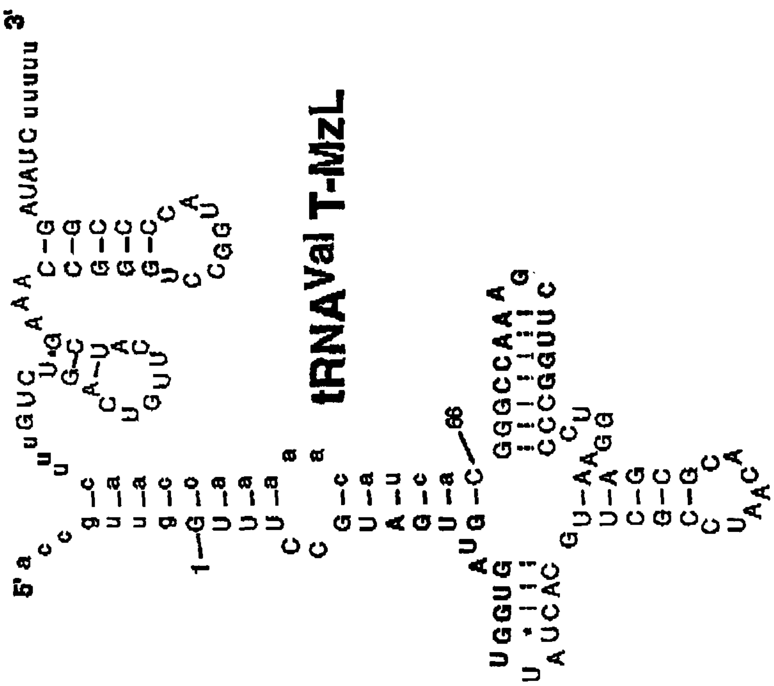

Fig.7 C

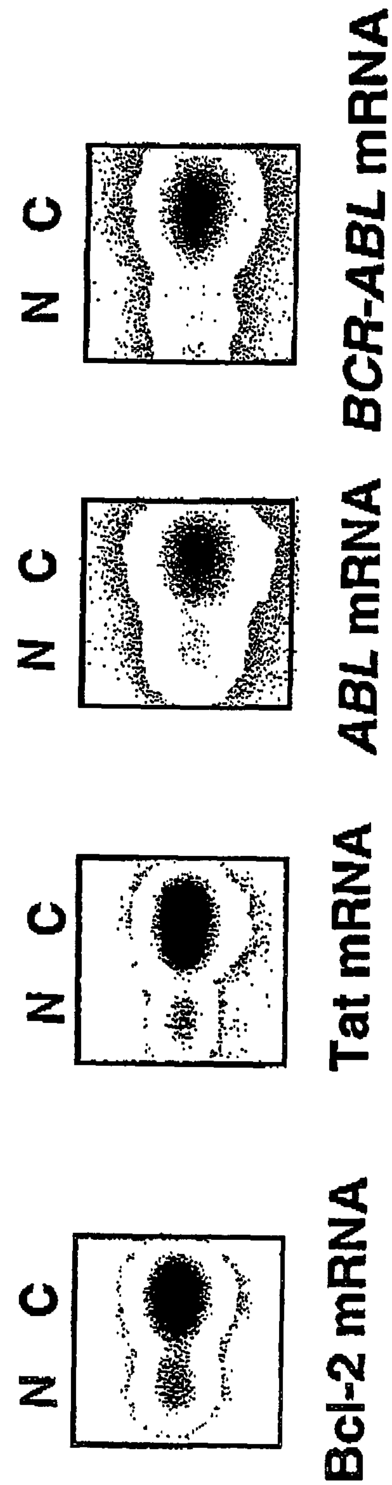

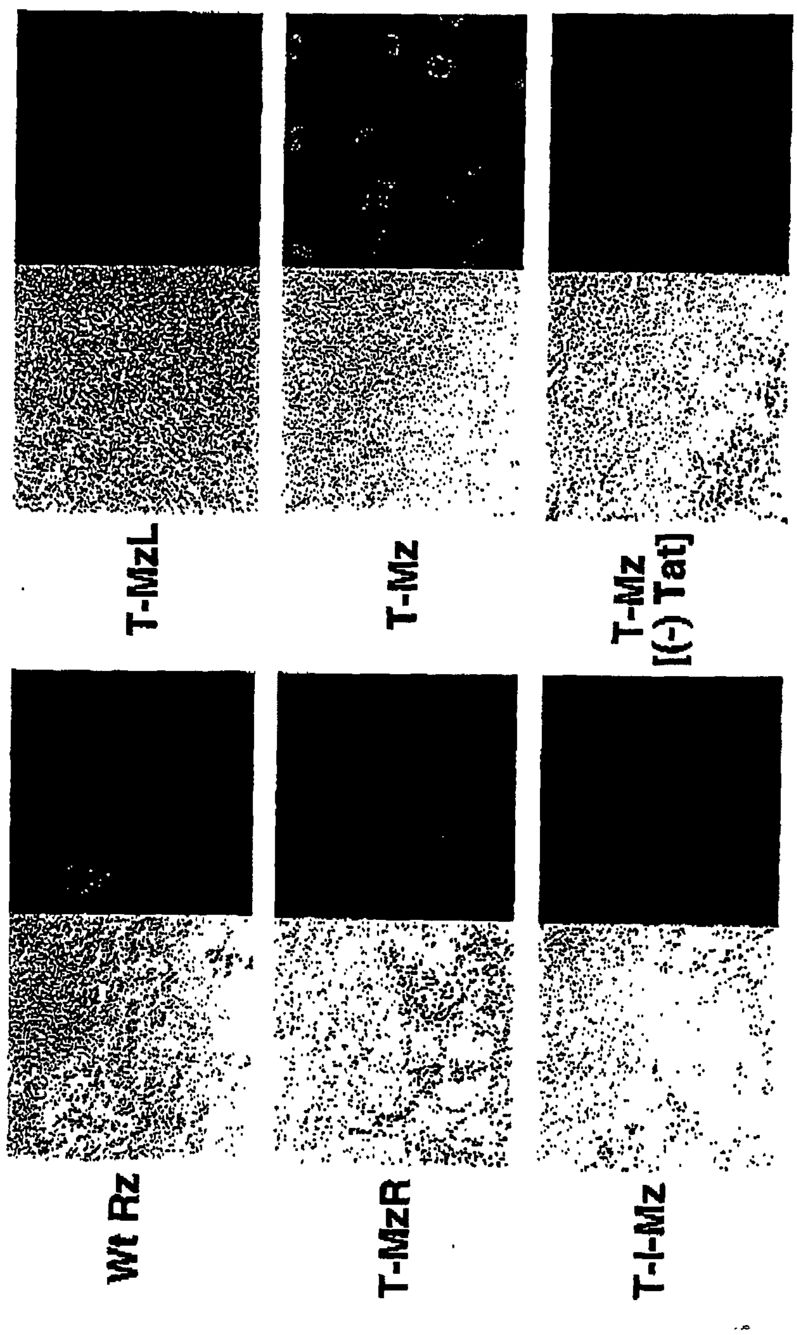


Fig.8 A

Fig.8 B

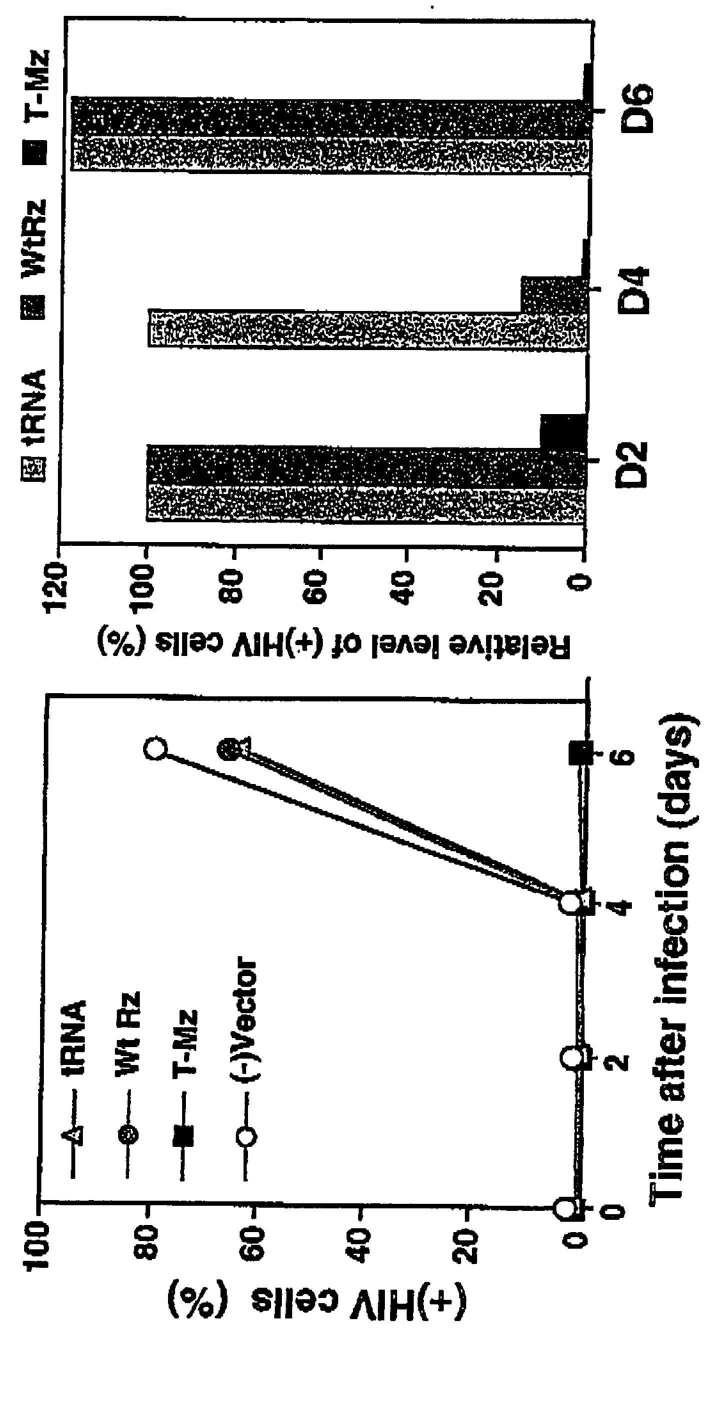

(+)HIV cells (%)
100
80
60
40
20
0
0
2
4
6
Time after infection (days)
tRNA
Wt Rz
T-Mz
(-)Vector
Relative level of (+)HIV cells (%)
120
100
80
60
40
20
0
D2
D4
D6
tRNA
WtRz
T-Mz

Fig.9

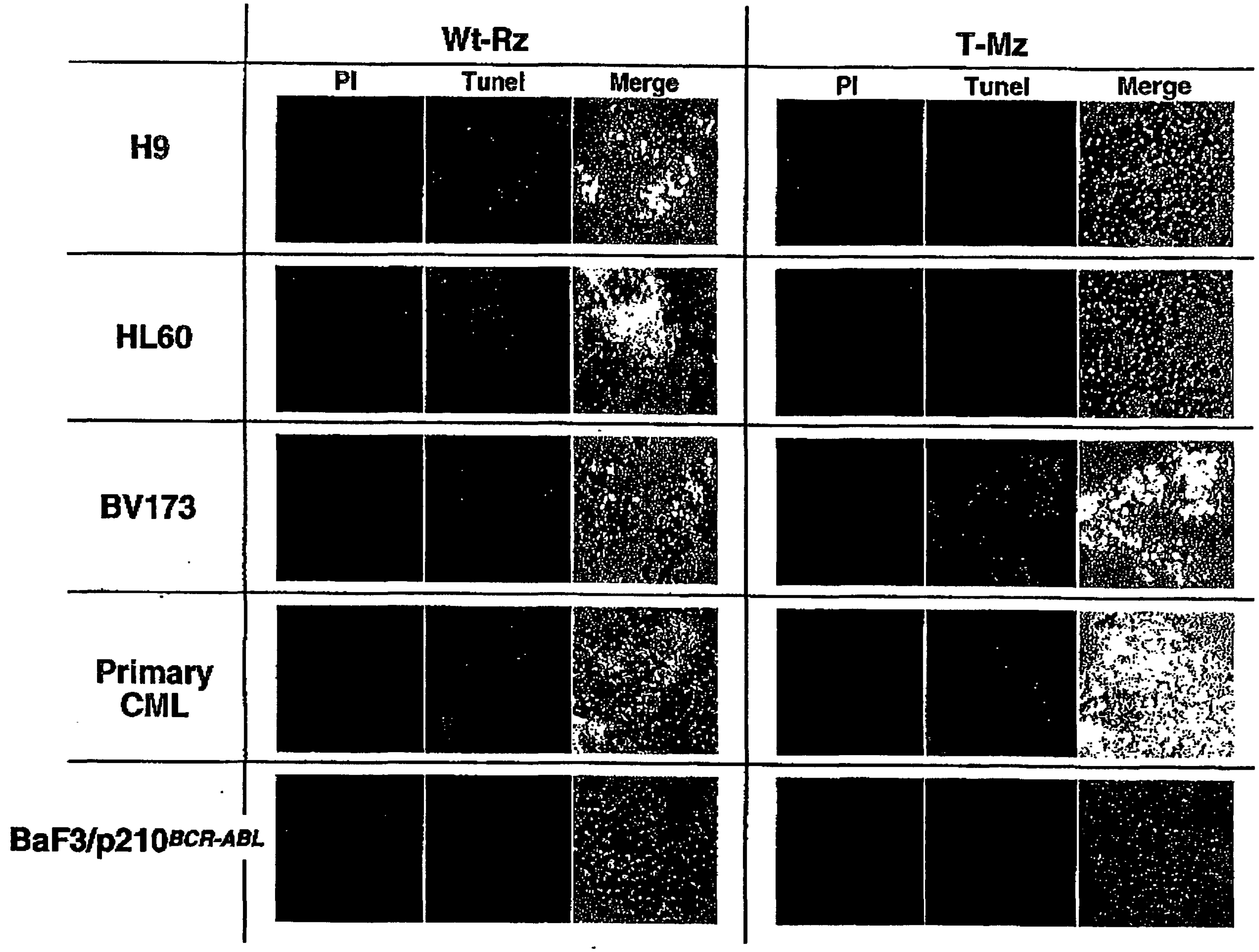
Wt-Rz
T-Mz
PI
Tunel
Merge
PI
Tunel
Merge
H9
HL60
BV173
Primary CML
BaF3/p210BCR-ABL

Fig.10 A

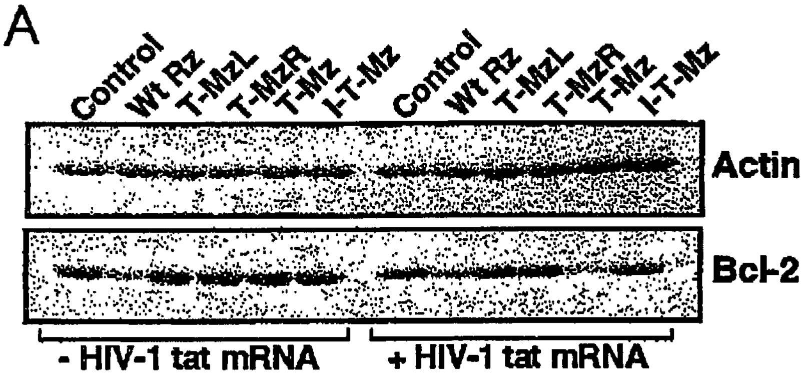


Fig.10 B

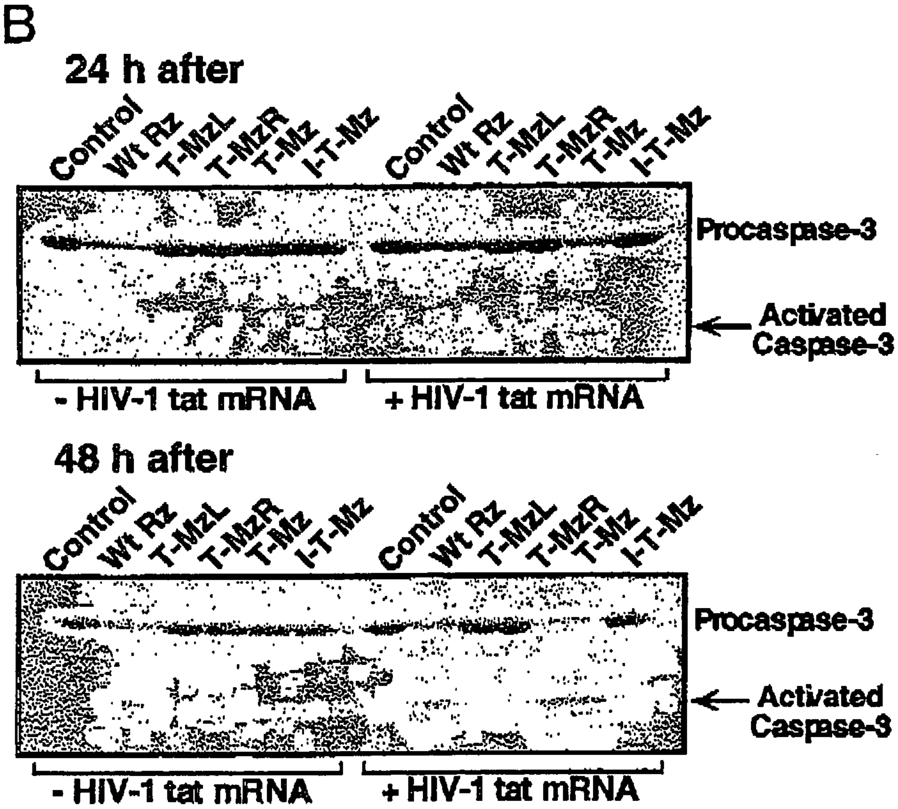

Fig.10 C

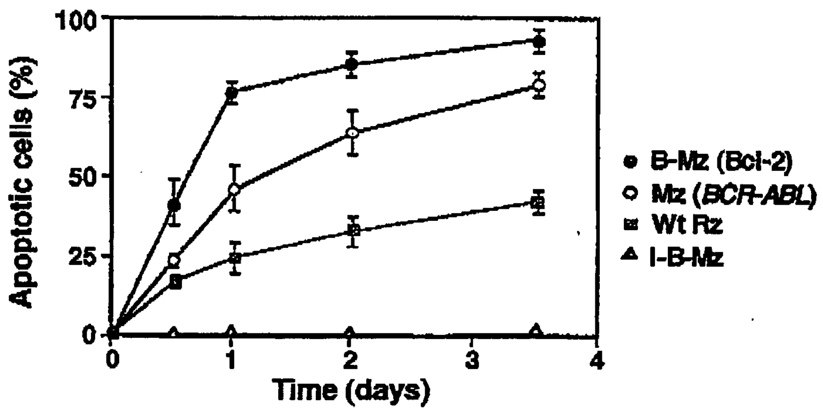


Fig.10 D

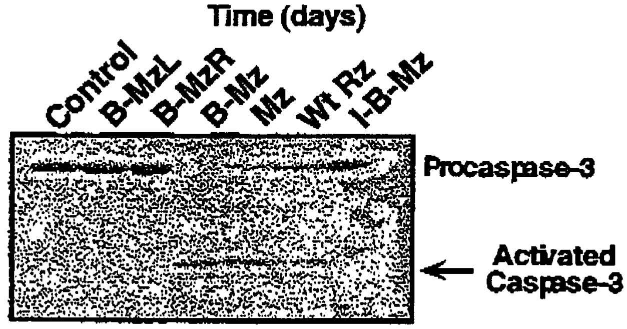


Fig.10 E

# SEQUENCE LISTING

<110> National Institute of Advanced Industrial Science and Technology

<120> Nucleic acid enzymes acquiring an activity for cleaving a target RNA by recognizing another molecule

<130>

<140>
<141>

<150> JP 2000-313320
<151> 2000-10-13

<160> 17

<170> PatentIn Ver. 2.0

<210> 1
<211> 32
<212> RNA
<213> Artificial Sequence

<220>
<223> Description of Artificial Sequence: maxizyme-constituting RNA mol ecule

<400> 1
gguccuggcc ugaugagagu gaugagcucu uc 32

<210> 2
<211> 27
<212> RNA
<213> Artificial Sequence

<220>
<223> Description of Artificial Sequence: maxizyme-constituting RNA mole
cule

<400> 2
gucugacugu ucaucgaaac cggucc 27

<210> 3
<211> 33
<212> RNA
<213> Artificial Sequence

<220>
<223> Description of Artificial Sequence: maxizyme-constituting RNA mole
cule

<400> 3
gguccuggcc ugaugagagu uauugauggu cag 33

<210> 4

<211> 29
<212> RNA
<213> Artificial Sequence

<220>
<223> Description of Artificial Sequence: maxizyme-constituting RNA molecule

<400> 4
gaagggcuuc uuucaucgaa accgggucc 29

<210> 5
<211> 88
<212> RNA
<213> Artificial Sequence

<220>
<223> Description of Artificial Sequence: $tRNA^{Val}$ promoter sequence

<400> 5
accguugguu uccguagugu agugguuauc acguucgccu aacacgcgaa agguccccgg 60
uucgaaaccg ggcacuacaa aaaccaac 88

<210> 6
<211> 33
<212> RNA
<213> Artificial Sequence

<220>

<223> Description of Artificial Sequence: ribozyme

<220>

<223> n is a, c, g or u.

<400> 6

nnnnncugau gaggccgaaa ggccgaaann nnn 33

<210> 7

<211> 24

<212> RNA

<213> Artificial Sequence

<220>

<223> Description of Artificial Sequence: left side sequence of maxizyme

<400> 7

cgaugaccug augagcgaaa cggc 24

<210> 8

<211> 24

<212> RNA

<213> Artificial Sequence

<220>

<223> Description of Artificial Sequence: right side sequence of maxizyme

<400> 8

cggggcugau gagcgaaacg uucc 24

<210> 9

<211> 13

<212> RNA

<213> Artificial Sequence

<220>

<223> Description of Artificial Sequence: substrate

<400> 9

gccgucguca ucg 13

<210> 10

<211> 11

<212> RNA

<213> Artificial Sequence

<220>

<223> Description of Artificial Sequence: substrate

<400> 10

gccgucccccc g 11

<210> 11

<211> 15

<212> RNA

<213> Artificial Sequence

<220>

<223> Description of Artificial Sequence: substrate

<400> 11

ggaacgucgu cgucg 15

<210> 12

<211> 40

<212> RNA

<213> Artificial Sequence

<220>

<223> Description of Artificial Sequence: wild type ribozyme

<400> 12

gguccuggcc ugaugaggcc gaaaggccga aaccgggucc 40

<210> 13

<211> 19

<212> RNA

<213> Artificial Sequence

<220>

<223> Description of Artificial Sequence: part of bcl-2 mRNA as a substrate

<400> 13

ggacccgguc gccaggacc 19

<210> 14

<211> 25

<212> RNA

<213> Artificial Sequence

<220>

<223> Description of Artificial Sequence: part of HIV tat mRNA

<400> 14

gaagagcuca ucagaacagu cagac 25

<210> 15

<211> 28

<212> RNA

<213> Artificial Sequence

<220>

<223> Description of Artificial Sequence: part of BCR-ABL mRNA

<400> 15

cugaccauca auaaggaaga agcccuuc 28

<210> 16

<211> 20

<212> RNA

<213> Artificial Sequence

<220>

<223> Description of Artificial Sequence: part of normal ABL mRNA

<400> 16

uuaucuggaa gaagcccuuc 20

<210> 17

<211> 138

<212> RNA

<213> Artificial Sequence

<220>

<223> Description of Artificial Sequence: $tRNA^{Val}$ T-MzL

<400> 17

accguugguu uccguagugu agugguuauc acguucgccu aacacgcgaa agguccccgg 60

uucgaaaccg ggcacuacaa aaaccaacuu ugucugacug uucaucgaaa ccgggguccgg 120

uaccccggau aucuuuuu 138